(19) Europäisches Patentamt
European Patent Office
Office européen des brevets

(11) EP 0 712 904 B1

(12) # EUROPÄISCHE PATENTSCHRIFT

(45) Veröffentlichungstag und Bekanntmachung des Hinweises auf die Patenterteilung:
**26.04.2000 Patentblatt 2000/17**

(51) Int Cl.[7]: **C09B 47/08**, C07F 9/6561, C09B 47/067, C09B 47/073, G11B 7/24

(21) Anmeldenummer: **95810569.4**

(22) Anmeldetag: **14.09.1995**

(54) **Mit Phosphorhaltigen Gruppen substituierte Phthalocyanine**

Phtalocyanines substituted with groups containing phosphorus

Phtalocyanines substituées par des groupes contenant du phosphore

(84) Benannte Vertragsstaaten:
**CH DE FR GB IT LI NL**

(30) Priorität: **23.09.1994 CH 290794**

(43) Veröffentlichungstag der Anmeldung:
**22.05.1996 Patentblatt 1996/21**

(73) Patentinhaber: **Ciba Specialty Chemicals Holding Inc.**
**4057 Basel (CH)**

(72) Erfinder:
- **Wolleb, Heinz, Dr.**
  **CH-1723 Marly (CH)**
- **Preiswerk, Hanspeter, Dr.**
  **CH-4127 Birsfelden (CH)**
- **Schmidhalter, Beat, Dr.**
  **CH-1735 Giffers (CH)**
- **Spahni, Heinz**
  **CH-4402 Frenkendorf (CH)**

(56) Entgegenhaltungen:
**EP-A- 0 391 415** **EP-A- 0 513 370**

- **SYNTHESIS, Nr. 9, 1984 STUTTGART-DE, Seiten 781-782, Y. XU ET AL. 'palladium-catalysed synthesis of alkylarylphenylphosphine oxides'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 712 904 B1

## Beschreibung

Die Erfindung betrifft mit Phosphorgruppen substituierte Phthalocyanine, ihre Herstellung und ihre Verwendung in Speicherschichten von optischen Informationsträgern.

Die Verwendung von Farbstoffen, die im nahen Infrarotbereich (NIR-Bereich) Strahlung absorbieren, zur Aufzeichnung von Informationen in WORM-Systemen (write once read many), ist seit längerem bekannt und zum Beispiel von M. Emmelius in Angewandte Chemie, Heft 11, Seiten 1475-1502 (1989) beschrieben. Durch die Laserbestrahlung in solchen Aufzeichnungsmaterialien kann die für die Aufzeichnung von Informationen in Form von Bits notwendige Änderung der Absorption durch physikalische Veränderungen (zum Beispiel durch Sublimation oder Diffusion) oder durch chemische Veränderungen (zum Beispiel Photochromie, Isomerisierungen oder thermische Zersetzung) erzielt werden.

Substituierte Phthalocyanine stellen eine wichtige Klasse von Farbstoffen für die Anwendung in solchen WORM-Systemen dar, da sie bei entsprechender peripherer Substitution, abhängig vom zentralen Metallatom, hohe NIR Absorptionen im Bereich von 700 nm bis 900 nm aufweisen.

In der EP-A-0373 643 werden mehrfach Alkoxy-substituierte und halogenierte Phthalocyanine beschrieben, deren langwelligen Absorptionsmaximum bei ca. 740 bis 820 nm liegt. Diese Verbindungen weisen entweder einen zu niedrigen Brechungsindex und eine zu niedrige Reflektivität bei 780 nm auf oder ihre Löslichkeiten in organischen Lösungsmitteln vermögen für "spin-coating" Verfahren nicht voll zu befriedigen.

Um diese Löslichkeitsprobleme insbesondere für Schleuderbeschichtungsverfahren zu überwinden, werden in der US-A-5 064 951 mit P(V) substituierte Naphthalocyanine vorgeschlagen, die eine breite Absorptionbande zwischen 700 nm und 800 nm aufweisen, wobei das Absorptionmaximum in der Regel auf der langwelligen Seite der Bande liegt. Diese Verbindungen haben in der Regel einen für die Praxis zu niedrigen Brechungsindex bei 780 nm und sind nicht ausreichend empfindlich.

In der EP-A-0513 370 werden lösliche, halogenierte, mit Alkoxy tetrasubstituierte Phthalocyanine beschrieben, deren Alkoxy Gruppen sterisch anspruchsvolle Reste enthalten. Die Absorptionsmaxima der dort beschriebenen Verbindungen liegen bei ca. 700 bis 730 nm und weisen einen molaren Extinktionskoeffizienten von >100 000 $l \cdot mol^{-1} \cdot cm^{-1}$ auf. Durch diese Eigenschaften kann man bei damit hergestellten optischen Speicherplatten einen ausreichend hohen Brechungsindex bei 780 nm und eine gute Empfindlichkeit erreichen.

Eine weitere Voraussetzung für eine hohe Empfindlichkeit ist, dass die Zersetzungstemperatur in einem günstigen Bereich liegt, der bevorzugt unterhalb ca. 300°C liegen sollte. Zur Herabsetzung des Zersetzungspunktes wird in EP-A-600 427 der Zusatz von Zersetzungsbeschleunigern wie beispielsweise Ferrocen vorgeschlagen. Die Anwesenheit von Zersetzungsbeschleunigern kann aber eine unerwünschte nachteilige Wirkung auf die Systeme haben, denen diese Additive zugegeben werden.

Neben den Absorptionseigenschaften und der photochemischen Stabilität müssen diese substituierten Phthalocyanine eine ausreichend gute Löslichkeit in organischen Lösungsmitteln aufweisen, da die Absorptionsschicht von optischen Speichermaterialien häufig in einem Schleuderbeschichtungsverfahren unter Verwendung von Lösungen aufgebracht werden. Als Lösungsmittel werden zum Beispiel in der EP-A-511 598 unpolare Lösungsmittel wie gesättigte oder ungesättigte Kohlenwasserstoffe empfohlen. Diese Löslichkeit in Kohlenwasserstoffen wird durch lipophile Substituenten, entsprechend den in der EP-A-0513 370 vorgeschlagenen Verbindungen, erreicht.

Es ist jedoch vom Herstellungsprozess von CD-Platten (compact disc) bekannt, dass solche Farbstofflösungen täglich frisch hergestellt werden müssen, da sich Farbstoffassoziate bilden können, die zur Rekristallisation und Ausfällung führen und letztlich unbrauchbare Produkte ergeben.

Ein weiteres Problem bei der Herstellung optischer Aufzeichnungsmaterialien ist die Kontrolle der Farbstoffmenge, die sich in Vertiefungen des Trägers absetzt. Um möglichst gleichmässige Filme zu bekommen, ist es daher häufig notwendig, der Farbstofflösung Verlaufshilfsmittel zuzusetzen, was wiederum die Reflexion und Empfindlichkeit beeinträchtigen kann.

Bei Verwendung polarerer Lösungsmittel, die stärkere van der Waals Kräfte oder gegebenenfalls Wasserstoffbrücken bilden können, kann das Problem der Kontrolle der Farbstoffmenge in den Vertiefungen auch ohne Verlaufshilfsmittel gelöst werden. Die optimalen Verlaufseigenschaften können durch Einstellung der Polarität des Lösungsmittels erreicht werden, insbesondere wenn Alkohole oder Ester mitverwendet werden können. Dadurch werden dem Hersteller optischer Aufzeichnungsmaterialien in der Fertigung mehr Möglichkeiten der Prozessoptimierung eröffnet.

Mit der vorliegenden Erfindung werden mit Phosphorgruppen substituierte Phthalocyanine bereitgestellt, welche viele überraschende Eigenschaften besitzen. So sind diese mit Phosphorgruppen substituierte Phthalocyanine gut löslich, sie neigen nicht zur Bildung von Assoziaten, haben einen Absorptionsmaximum bei ca. 700 bis 735 nm mit einen hohen molaren Extinktionskoeffizienten von >100 000 l/Mol.cm und weisen eine niedrigere Zersetzungstemperatur auf als vergleichbare Phthalocyanine, welche nicht mit Phosphorgruppen substituiert sind. Die Struktur der Absorptionsbande ist relativ schmal und der Brechungsindex bei 780 nm hoch, wodurch ein guter Kontrast zwischen dem

eingeschriebenen und dem nicht eingeschriebenen Zustand erhalten werden kann. Die Produkte haben vorteilhafte tiefe Schmelzpunkte oder fallen in fester Form amorph an. Durch die Einführung einer Phosphorgruppe lässt sich die Polarität der Verbindungen in einem weiten Bereich beeinflussen, was für die Auswahl des Lösungsmittels mehr Möglichkeiten eröffnet. Die Lagerstabilität der Lösungen ist gut, so dass grössere Mengen an Vorratslösungen ohne Assoziation und Rekristallisation über einen längeren Zeitraum aufbewahrt werden können und eine wirtschaftliche Verarbeitung möglich ist. Werden die erfindungsgemässen mit Phosphorgruppen substituierten Phthalocyanine zusammen mit einem Zersetzungsbeschleuniger eingesetzt, so kann gegebenenfalls die Menge des letzteren reduziert werden.

Ein Gegenstand der Erfindung sind Phthalocyanine und deren Metallkomplexe von divalenten Metallen oder divalenten Oxometallen, die dadurch gekennzeichnet sind, dass sie mindestens einen phosphorhaltigen Substituenten über das Phosphoratom am peripheren Kohlenstoffgerüst gebunden enthalten.

Die Verbindungen entsprechen bevorzugt der allgemeinen Strukturformel $(G)_k$-A-$(Q)_l$, wobei A ein Phthalocyanin oder seinen Metallkomplex mit einem divalenten Metall oder divalenten Oxometall bedeutet, G für einen phosphorhaltigen Substituenten und Q für weitere Substituenten, wie sie nachfolgend definiert sind, steht. Der Index k bedeutet eine Zahl von 1 bis 16 und l bedeutet unabhängig 0 oder eine Zahl von 1 bis 15, unter der Bedingung, dass die Summe von k und l eine Zahl kleiner oder gleich 16 ergibt.

Bevorzugt sind 1 bis 4 und besonders bevorzugt 1 bis 3 phosphorhaltige Substituenten am peripheren Kohlenstoffgerüst gebunden enthalten.

Bevorzugt enthält das periphere Kohlenstoffgerüst insgesamt 4-16, besonders bevorzugt insgesamt 5-10 Substituenten.

Für die divalenten Metallatome oder divalenten Oxometalle der Phthalocyanin Metallkomplexe gelten die nachstehend aufgeführten Bevorzugungen.

Bevorzugt wird der phosphorhaltige Substituent aus der Gruppe bestehend aus $-PR_1R_2$, $-P(X)R_3$, -P(X)(Y), $-PZR_4R_5$, $-PZ(X)R_6$, -PZ(X)(Y) ausgewählt, wobei

$R_1$, $R_2$, $R_3$, $R_4$, $R_5$ und $R_6$ unabhängig voneinander unsubstituiertes oder mit -OH, Halogen, $C_1$-$C_{12}$-Alkoxy, $-COOR_7$, -CN, Phenyl, Naphthyl substituiertes lineares oder verzweigtes $C_1$-$C_{12}$-Alkyl, $C_3$-$C_{12}$-Alkenyl oder $C_3$-$C_{12}$-Alkinyl, unsubstituiertes oder mit -OH, Halogen, $C_1$-$C_{12}$-Alkoxy, $-COOR_7$, -CN substituiertes $C_3$-$C_8$-Cycloalkyl, Phenyl oder Naphthyl bedeuten;

X und Y unabhängig voneinander $-OR_8$, $-SR_9$ oder $-NR_{10}R_{11}$ sind;

Z für O, S, Se oder Te steht;

$R_7$ lineares oder verzweigtes $C_1$-$C_{12}$-Alkyl, $C_3$-$C_{12}$-Alkenyl oder $C_3$-$C_{12}$-Alkinyl ist;

$R_8$, $R_9$ unabhängig voneinander ein Alkalimetall-Kation, $NH_4^+$, Wasserstoff, unsubstituiertes oder mit -OH, Halogen, $C_1$-$C_{12}$-Alkoxy, $-COOR_7$, -CN, Phenyl, Naphthyl substituiertes lineares oder verzweigtes $C_1$-$C_{12}$-Alkyl, $C_3$-$C_{12}$-Alkenyl oder $C_3$-$C_{12}$-Alkinyl, unsubstituiertes oder mit -OH, Halogen, $C_1$-$C_{12}$-Alkoxy, $-COOR_7$, -CN substituiertes $C_3$-$C_8$-Cycloalkyl, Phenyl oder Naphthyl sind; und

$R_{10}$, $R_{11}$ unabhängig voneinander Wasserstoff oder unsubstituiertes oder mit -OH, Halogen, $C_1$-$C_{12}$-Alkoxy, $-COOR_7$, -CN, Phenyl, Naphthyl substituiertes lineares oder verzweigtes $C_1$-$C_{12}$-Alkyl, $C_3$-$C_{12}$-Alkenyl oder $C_3$-$C_{12}$-Alkinyl bedeuten, oder unsubstituiertes oder mit -OH, Halogen, $C_1$-$C_{12}$-Alkoxy, $-COOR_7$, -CN substituiertes $C_3$-$C_8$-Cycloalkyl, Phenyl oder Naphthyl sind.

Beispiele für lineare oder verzweigte $C_1$-$C_{12}$-Alkylreste sind z.B. Methyl, Ethyl sowie die verschiedenen Stellungsisomeren von Propyl, Butyl, Pentyl, Hexyl, Heptyl, Octyl, Nonyl, Decyl, Undecyl oder Dodecyl.

Beispiele für $C_3$-$C_{16}$-Alkenylreste sind Propenyl, Butenyl, Pentenyl, Hexenyl, Heptenyl, Octenyl, Nonenyl, Decenyl, Undecenyl oder Dodecenyl mit ihren verschiedenen Stellungsisomeren.

Beispiele für $C_3$-$C_{16}$-Alkinylreste sind Propinyl, Butinyl, Pentinyl, Hexinyl, Heptinyl, Octinyl, Noninyl, Decinyl, Undecinyl, Dodecinyl, Tridecinyl, Tetradecinyl, Pentadecinyl oder Hexadecinyl mit ihren verschiedenen Stellungsisomeren.

Halogen steht zum Beispiel für Fluor, Chlor, Brom, oder Jod.

$C_1$-$C_{12}$-Alkoxy bedeutet zum Beispiel Methoxy, Ethoxy sowie die verschiedenen Stellungsisomeren von Propoxy, Butoxy, Pentoxy, Hexyloxy, Heptyloxy, Octyloxy, Nonyloxy, Decyloxy, Undecyloxy oder Dodecyloxy.

$C_3$-$C_8$-Cycloalkyl steht beispielsweise für Cyclopropyl, Dimethylcyclopropyl, Cyclobutyl, Cyclopentyl, Methylcyclopentyl, Cyclohexyl, Cycloheptyl oder Cyclooctyl.

Alkalimetallkationen sind beispielsweise $Li^+$, $Na^+$ oder $K^+$.

Bevorzugt steht Z für O oder S, ganz besonders bevorzugt für O.

$R_1$, $R_2$, $R_3$, $R_4$, $R_5$ und $R_6$ bedeuten unabhängig voneinander bevorzugt lineares oder verzweigtes unsubstituiertes $C_1$-$C_{12}$-Alkyl, $C_3$-$C_{12}$-Alkenyl, $C_3$-$C_{12}$-Alkinyl, $C_3$-$C_8$-Cycloalkyl, Phenyl oder Naphthyl.

Besonders bevorzugt sind $R_1$, $R_2$, $R_3$, $R_4$, $R_5$ und $R_6$ unabhängig voneinander lineares oder verzweigtes

unsubstituiertes $C_1$-$C_8$-Alkyl, $C_3$-$C_8$-Alkenyl, $C_3$-$C_8$-Alkinyl, $C_3$-$C_8$-Cycloalkyl oder Phenyl.

$R_7$ bedeutet bevorzugt lineares oder verzweigtes unsubstituiertes $C_1$-$C_{12}$-Alkyl.

Bevorzugt sind $R_8$ und $R_9$ unabhängig voneinander ein Alkalimetall-Kation, $NH4^+$, Wasserstoff, lineares oder verzweigtes unsubstituiertes $C_1$-$C_{12}$-Alkyl, $C_3$-$C_{12}$-Alkenyl oder $C_3$-$C_{12}$-Alkinyl, $C_3$-$C_8$-Cycloalkyl oder Phenyl und besonders bevorzugt $C_1$-$C_6$-Alkyl, $C_3$-$C_8$-Cycloalkyl oder Phenyl.

Bevorzugt stehen $R_{10}$ und $R_{11}$ unabhängig voneinander für lineares oder verzweigtes unsubstituiertes $C_1$-$C_{12}$-Alkyl, $C_3$-$C_{12}$-Alkenyl oder $C_3$-$C_{12}$-Alkinyl, $C_3$-$C_8$-Cycloalkyl oder Phenyl und besonders bevorzugt für lineares oder verzweigtes $C_1$-$C_6$-Alkyl.

Eine bevorzugte Untergruppe von Phthalocyaninen und deren Metallkomplexen sind Verbindungen der Formel I

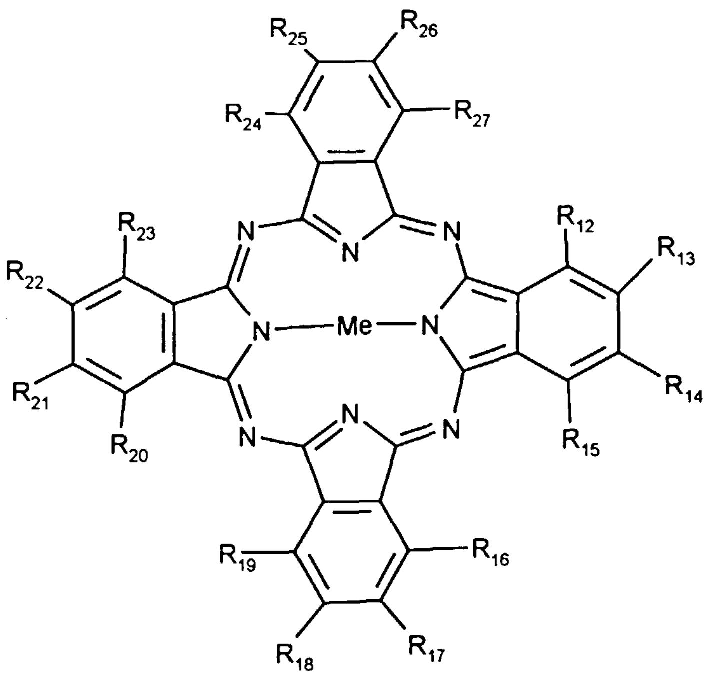

(I),

worin Me ein divalentes Metallatom oder ein divalentes Oxometall oder 2 Wasserstoffatome bedeutet,

$R_{12}$ bis $R_{27}$ unabhängig voneinander-$PR_1R_2$, -$P(X)R_3$, -P(X)(Y), -$PZR_4R_5$, -$PZ(X)R_6$, -PZ(X)(Y), Wasserstoff, -OH, Halogen, unsubstituiertes oder mit -OH, Halogen, $C_1$-$C_{12}$-Alkoxy, -$COOR_7$, -CN, Phenyl, Naphthyl substituiertes lineares oder verzweigtes $C_1$-$C_{12}$-Alkyl, $C_3$-$C_{12}$-Alkenyl, $C_3$-$C_{12}$-Alkinyl, $C_1$-$C_{12}$-Alkoxy, oder $C_1$-$C_{12}$-Alkylthio, unsubstituiertes oder mit -OH, Halogen, $C_1$-$C_{12}$-Alkoxy, -$COOR_7$, -CN substituiertes $C_3$-$C_8$-Cycloalkyl, Phenyl, Naphthyl, Phenoxy, Thiophenyl, Naphthoxy oder Thionaphthyl, -$COOR_{28}$, -$CONR_{29}R_{30}$, -$SO_3R_{31}$, -$SO_2NR_{32}R_{33}$, -$SiR_{34}R_{35}R_{36}$, -$NR_{37}R_{38}$ bedeuten, worin $R_{28}$ bis $R_{38}$ unabhängig voneinander Wasserstoff, unsubstituiertes oder mit -OH, Halogen, -$COOR_7$, -CN, Phenyl, Naphthyl substituiertes lineares oder verzweigtes $C_1$-$C_{12}$-Alkyl, $C_3$-$C_{12}$-Alkenyl oder $C_3$-$C_{12}$-Alkinyl, unsubstituiertes oder mit -OH, Halogen, $C_1$-$C_{12}$-Alkoxy, -$COOR_7$, -CN substituiertes $C_3$-$C_8$-Cycloalkyl, Phenyl oder Naphthyl sind; mit der Maßgabe, dass mindestens einer der Substituenten $R_{12}$ bis $R_{27}$ ein phosphorhaltiger Substituent ausgewählt aus der Gruppe bestehend aus -$PR_1R_2$, -$P(X)R_3$, -P(X)(Y), -$PZR_4R_5$, -$PZ(X)R_6$, -PZ(X)(Y) ist, wobei die Bedeutung von $R_1$-$R_6$, X, Y und Z sowie deren Bevorzugungen vorstehend angegeben sind.

Bevorzugt ist Me als divalentes Metallatom Cu(II), Zn(II), Fe(II), Ni(II), Ru(II), Rh(II), Pd(II), Pt(II), Mn(II), Mg (II), Be(II), Ca(II), Ba(II), Cd(II), Hg(II), Sn(II), Co(II) oder Pb(II), oder als divalentes Oxometall VO, MnO oder TiO. Ganz besonders bevorzugt sind Zn(II), Sn(II), Cu(II), Ni(II), Co(II), Pb(II), Pd(II) oder V(O).

$R_{12}$ bis $R_{27}$ sind bevorzugt unabhängig voneinander Wasserstoff, Halogen, lineares oder verzweigtes unsubstituiertes $C_1$-$C_{12}$-Alkyl, $C_3$-$C_{12}$-Alkenyl, $C_3$-$C_{12}$-Alkinyl, $C_1$-$C_{12}$-Alkoxy oder $C_1$-$C_{12}$-Alkylthio, Phenyl, Phenoxy oder Thiophenyl.

Besonders bevorzugt sind $R_{12}$ bis $R_{27}$ unabhängig voneinander Wasserstoff, Halogen, lineares oder verzweigtes unsubstituiertes $C_4$-$C_{10}$-Alkyl, $C_4$-$C_{10}$-Alkenyl, $C_4$-$C_{10}$-Alkinyl oder $C_4$-$C_{10}$-Alkoxy und ganz besonders bevorzugt Wasserstoff, Br, Cl oder lineares oder verzweigtes unsubstituiertes $C_4$-$C_{10}$-Alkoxy.

Bevorzugt sind $R_{28}$ bis $R_{38}$ unabhängig voneinander Wasserstoff, lineares oder verzweigtes unsubstituiertes $C_1$-$C_{12}$-Alkyl, $C_3$-$C_{12}$-Alkenyl oder $C_3$-$C_{12}$-Alkinyl, $C_3$-$C_8$-Cycloalkyl, Phenyl oder Naphthyl, besonders bevorzugt Was-

serstoff oder $C_1$-$C_{12}$-Alkyl.

In einer bevorzugten Untergruppe von Metallkomplexen von Phthalocyaninen bedeuten $R_1$ bis $R_6$ unabhängig voneinander $C_1$-$C_4$-Alkyl oder Phenyl, X und Y unabhängig voneinander -$OR_8$ oder -$NR_{10}R_{11}$, Z steht für O, Me für Pd, V(O), Cu oder Ni, insbesondere Pd, V(O) oder Cu, und $R_{12}$ bis $R_{27}$ sind unabhängig voneinander Wasserstoff, Br oder lineares oder verzweigtes unsubstituiertes $C_4$-$C_{10}$-Alkoxy.

Die Gesamtzahl der Substituenten am peripheren Kohlenstoffgerüst beträgt bevorzugt 4 bis 10.

Besonders bevorzugt sind Metallkomplexe von Phthalocyaninen bei denen die phosphorhaltigen Substituenten Reste der Formeln II-VI darstellen:

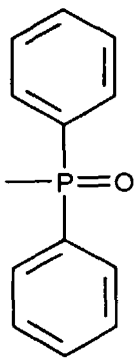 (II), 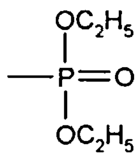 (III), 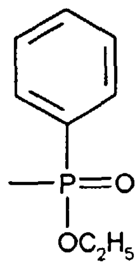 (IV), 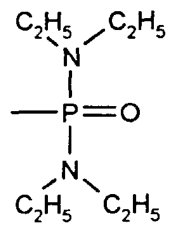 (V), 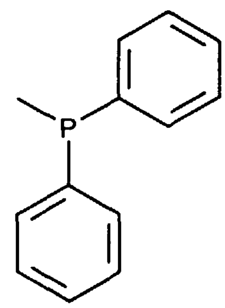 (VI).

Die erfindungsgemässen Verbindungen können in Analogie zu an sich bekannten Verfahren hergestellt werden. Es können zum Beispiel Phosphorgruppen enthaltende Phthalocyanine durch Kondensation von Phosphorgruppen enthaltenden Phthalsäuren, Phthalsäureanhydriden, Phthalimiden, Phthalsäurediamiden, Diiminodihydroisoindolinen oder Phthalodinitrilen nach allgemein bekannten Synthesemethoden hergestellt werden, zum Beispiel wie von F. H. Moser, A. L. Thomas, The Phthalocyanines Volume II, CRC Press, Inc., Boca Raton, Florida, 1983 beschrieben.

Ein weiterer Gegenstand der Erfindung ist ein Verfahren zur Herstellung von Phthalocyaninen und deren divalenten Metallkomplexen oder divalenten Oxometallkomplexen durch Umsetzung eines einen phosphorhaltigen Substituenten enthaltenden Phthalodinitrils oder Diiminodihydroisoindolins, oder eines Gemisches von Phthalodinitrilen oder Diiminodihydroisoindolinen, von welchen mindestens eines einen phosphorhaltigen Substituenten enthält, welche gegebenenfalls unabhängig voneinander weitere Substituenten enthalten können, gegebenenfalls in Gegenwart eines Metallsalzes oder Oxometallsalzes.

Bevorzugt ist ein Verfahren zur Herstellung von Phthalocyaninen und deren Metallkomplexen der Formel I durch Umsetzung einer Verbindung der Formel VII oder VIII,

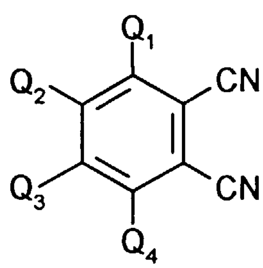 (VII), 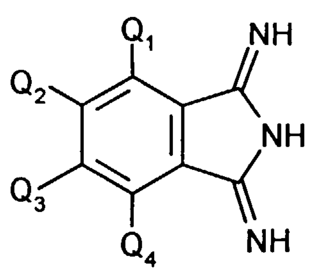 (VIII),

oder eines Gemisches von 2 bis 4 dieser Verbindungen,
gegebenenfalls in Gegenwart eines Metallsalzes, wobei $Q_1$ bis $Q_4$ unabhängig voneinander -$PR_1R_2$, -P(X)$R_3$, -P(X)(Y), -$PZR_4R_5$,-PZ(X)$R_6$, -PZ(X)(Y), Wasserstoff, -OH, Halogen, unsubstituiertes oder mit -OH, Halogen, $C_1$-$C_{12}$-Alkoxy, -$COOR_7$, -CN, Phenyl, Naphthyl substituiertes $C_1$-$C_{12}$-Alkyl, $C_3$-$C_{12}$-Alkenyl, $C_3$-$C_{12}$-Alkinyl, $C_1$-$C_{12}$-Alkoxy, oder $C_1$-$C_{12}$-Alkylthio, unsubstituiertes oder mit -OH, Halogen, $C_1$-$C_{12}$-Alkoxy, -$COOR_7$, -CN substituiertes $C_3$-$C_8$-Cycloalkyl, Phenyl, Naphthyl, Phenoxy, Thiophenyl, Naphthoxy oder Thionaphthyl, -$COOR_{28}$, -$CONR_{29}R_{30}$, -$SO_3R_{31}$, -$SO_2NR_{32}R_{33}$, -$SiR_{34}R_{35}R_{36}$, -$NR_{37}R_{38}$ bedeuten, worin
$R_1$ bis $R_7$ und $R_{28}$ bis $R_{38}$ die vorstehend angegebene Bedeutung haben,
mit der Maßgabe, dass mindestens eine Verbindung der Formel VII oder VIII mindestens einen Substituent $Q_1$ bis $Q_4$

trägt, der aus der Gruppe phosphorhaltiger Substituenten bestehend aus $-PR_1R_2$, $-P(X)R_3$, -P(X)(Y), $-PZR_4R_5$, -PZ(X) $R_6$, -PZ(X)(Y) ausgewählt ist, wobei $R_1$-$R_6$, X, Y und Z die vorstehend angegebene Bedeutung haben.

Man erhält dabei je nach der Zahl der eingesetzten Komponenten ein Gemisch aus verschiedenen $\alpha$ oder $\beta$ substituierten stellungsisomeren Phthalocyaninen.

Für die Metalle oder Oxometalle in den Salzen gelten die vorstehend für Me bezeichneten Definitionen und Bevorzugungen.

Bevorzugt werden Metallsalze eingesetzt bei denen das Anion von einer ein- oder zweibasigen anorganischen Säure, einer $C_1$-$C_{12}$-Carbonsäure oder einem $C_5$-$C_{12}$-β-Diketon stammt.

Als anorganische Säuren kommen vor allem HCl, HBr, $H_2SO_4$, $HNO_3$ oder $HClO_4$ in Frage. Geeignete $C_1$-$C_{12}$-Carbonsäuren sind zum Beispiel Ameisensäure, Essigsäure, Propionsäure, die verschiedenen Isomeren der Buttersäure, Valeriansäure oder Capronsäure. Als $C_5$-$C_{12}$-β-Diketone kommen zum Beispiel Acetylaceton, Hexan-2,4-Dion, Heptan-3,5-Dion, Heptan-2,4-Dion, die verschiedenen Stellungsisomeren Octan-, Nonan-, Decan-, Undecan- und Dodecan-β-Dione in Frage.

Besonders bevorzugt verwendet man als Metallsalz $Pd(II)Cl_2$, $Cu(II)Cl_2$, $Zn(II)Cl_2$, $Ni(II)Cl_2$, Cu(II)-acetylacetonat oder V(III)-acetylacetonat.

Ganz besonders bevorzugt werden $Pd(II)Cl_2$, $Cu(II)Cl_2$ oder $Ni(II)Cl_2$ eingesetzt.

Die Kondensationsreaktion wird bevorzugt in einem Lösungsmittelgemisch aus Nitrobenzol, Nitrotoluol oder Nitroxylol und mindestens einer äquimolaren Menge Harnstoff, bezogen auf die Verbindungen der Formel VII oder VIII, durchgeführt.

Die erfindungsgemässen Verbindungen können auch durch Umsetzung von mit einer Abgangsgruppe substituierten Phthalocyaninen oder deren Metallkomplexen mit einer Phosphorverbindung in Gegenwart eines Metallkatalysators in Analogie zu allgemein bekannten Methoden hergestellt werden. Geeignete solche Methoden können zum Beispiel Houben-Weyl, Methoden der Organischen Chemie, Bände 12/1, 12/2, E1 und E2, Georg Thieme Verlag, Stuttgart, oder insbesondere der Publikation von Y. Xu et al., Synthesis 1984/9, 781-2 (1984) entnommen werden, wobei letztere den Verbrauch von Tetrakis[triphenylphosphin]-palladium als Katalysator in Toluol als Lösungsmittel bescheibt.

Bevorzugt werden dabei halogenierte Phthalocyanine, deren Halogengruppe als Abgangsgruppe dient, mit Phosphorverbindungen umgesetzt. Ganz überraschend wurde gefunden, dass bei dieser Umsetzung eine markant bessere Ausbeute erzielt werden kann, falls sie in Gegenwart eines Palladium(II)-Komplexes in einem sehr polaren Lösungsmittel durchgeführt wird.

Als polares Lösungsmittel wird dabei bevorzugt Dimethylformamid (DMF) verwendet. Andere geeignete sehr polare Lösungsmittel sind zum Beispiel solche mit einer Dielektrizitätskonstante $\varepsilon \geq 25$, beispielsweise Ethylencarbonat, Dimethylsulfoxid, Sulfolan oder insbesondere Amide wie Formamid, N-Methyl-formamid, Acetamid, N-Methyl-acetamid, N,N'-Dimethyl-acetamid, N-Methyl-propionamid, Pyrrolidon, N-Methyl-pyrrolidon (NMP) oder 1,1,2,2-Tetramethylharnstoff.

Als Palladium(II)-Komplex wird bevorzugt $PdCl_2$ / Triphenylphosphin verwendet. Andere geeignete Katalysatoren wie beispielsweise Tetrakis-(triphenylphosphin)-palladium sind an sich bekannt und in der oben erwähnten Literatur beschrieben. Vorteilhaft können auch Phosphorverbindungen von anderen Übergangsmetallen eingesetzt werden.

Ein weiterer Gegenstand der Erfindung ist daher ein Verfahren zur Herstellung von Phthalocyaninen oder deren Metallkomplexen durch Umsetzung eines Phthalocyanins oder seines Metallkomplexes, das mindestens einen Halogensubstituenten als Abgangsgruppe enthält, mit einer phosphorhaltigen Verbindung.

Bevorzugt ist ein Verfahren zur Herstellung von Phthalocyaninen oder deren Metallkomplexen der Formel I durch Umsetzung eines Phthalocyanins oder seines Metallkomplexes, das mindestens einen Halogensubstituenten als Abgangsgruppe enthält, mit einer phosphorhaltigen Verbindung, ausgewählt aus der Gruppe bestehend aus $PR_1R_2R_3$, $P(X)_2R_3$, $P(X)_2(Y)$, $P(X)R_1R_2$, $P(X)_2OH$, in Gegenwart eines Katalysators, wobei $R_1$ bis $R_6$, X, Y und Z die vorstehend angegebene Bedeutung haben.

Besonders bevorzugt ist ein Verfahren zur Herstellung von Phthalocyaninen oder deren Metallkomplexen der Formel I durch Umsetzung eines Phthalocyanins oder seines Metallkomplexes, das mindestens einen Halogensubstituenten als Abgangsgruppe enthält, mit einer phosphorhaltigen Verbindung in Dimethylformamid und in Gegenwart von Palladium(II)-chlorid und Triphenylphosphin.

Die entsprechenden Zwischenprodukte, die phosphorsubstituierten Phthalsäuren, Phthalsäureanhydride, Phthalimide, Phthalsäurediamide, Diiminodihydroisoindoline und Phthalodinitrile können durch Substitutionsreaktionen von entsprechend funktionalisierten Edukten, bevorzugt Bromiden, mit Phosphorverbindungen nach allgemein bekannten Methoden z. B. nach Houben-Weyl, Methoden der Organischen Chemie, Bände 12/1, 12/2, E1 und E2, Georg Thieme Verlag, Stuttgart) hergestellt werden.

Gegenstand der Erfindung ist auch ein Material zur optischen Aufzeichnung und Speicherung von Information, bei dem auf einem transparenten, dielektrischen Trägermaterial (1) mindestens eine Speicherschicht (2) einer Phthalocyaninverbindung oder deren Metallkomplexe nach Anspruch 1 aufgebracht ist.

Die Dicke der Speicherschicht (2) kann 10 nm bis 1000 nm betragen.

Das Material kann eine zusätzliche Reflexionsschicht enthalten, die zum Beispiel auf der Speicherschicht aufgebracht ist. Sie kann eine Dicke von 10 nm bis 50 µm aufweisen.

Als reflektierendes Material für die Reflexionsschicht eignen sich besonders Metalle, die die zur Aufzeichnung und Wiedergabe verwendete Laserstrahlung gut reflektieren, zum Beispiel die Metalle der dritten, vierten und fünften Hauptgruppe und den Nebengruppen des periodischen Systems der chemischen Elemente. Besonders geeignet sind Al, In, Sn, Pb, Sb, Bi, Cu, Ag, Au, Zn, Cd, Hg, Sc, Y, La, Ti, Zr, Hf, V, Nb, Ta, Cr, Mo, W, Fe, Co, Ni, Ru, Rh, Pd, Os, Ir, Pt und die Lanthanidenmetalle Ce, Pr, Nd, Pm, Sm, Eu, Gd, Tb, Dy, Ho, Er, Tm, Yb und Lu. Besonders bevorzugt ist aus Gründen der hohen Reflektivität und leichten Herstellbarkeit eine Reflexionsschicht aus Aluminium oder Gold.

Die je nach Schichtaufbau oberste Schicht, zum Beispiel die Reflexionsschicht oder die Schicht aus einem Phthalocyanin oder seinem Metallkomplex, wird zweckmässig mit einer Schutzschicht versehen, die eine Dicke von 0,1 bis 100 µm, bevorzugt 0,1 bis 50 µm und besonders bevorzugt 0,5 bis 15 µm aufweisen kann. Als Schutzmaterial eignen sich hauptsächlich Kunststoffe, die in dünner Schicht entweder direkt oder mit Hilfe von Haftschichten auf den Träger oder die oberste Schicht aufgetragen sind. Man wählt zweckmässig mechanisch und thermisch stabile Kunststoffe mit guten Oberflächeneigenschaften, die noch modifiziert, zum Beispiel beschrieben werden können. Es kann kann sich sowohl um duroplastische wie auch thermoplastische Kunststoffe handeln. Bevorzugt sind strahlungsgehärtete (zum Beispiel mit UV-Strahlung) Schutzschichten, die besonders einfach und wirtschaftlich herstellbar sind. Strahlungshärtbare Materialien sind in grosser Vielzahl bekannt. Beispiele für strahlungshärtbare Monomere und Oligomere sind Acrylate und Methacrylate von Diolen, Triolen und Tetrolen, Polyimide aus aromatischen Tetracarbonsäuren und aromatischen Diaminen mit $C_1$-$C_4$-Alkylgruppen in mindestens zwei Orthostellungen der Aminogruppen, und Oligomere mit Dialkyl-, zum Beispiel Dimethylmaleinimidylgruppen.

Geeignete Träger sind zum Beispiel Metalle, Metallegierungen, Gläser, Mineralien, Keramiken und duroplastische oder thermoplastische Kunststoffe. Der Träger kann eine Dicke von 0,01 mm bis 1 cm, bevorzugt 0,1 mm bis 0,5 cm aufweisen. Bevorzugte Träger sind Gläser und homo- oder copolymere Kunststoffe. Geeignete Kunststoffe sind zum Beispiel thermoplastische Polycarbonate, Polyamide, Polyester, Polyacrylate und Polymethacrylate, Polyurethane, Polyolefine, Polyvinylchlorid, Polyvinylidenfluorid, Polyimide, duroplastische Polyester und Epoxidharze.

Die erfindungsgemäss zu verwendenden Aufzeichnungsmaterialien können nach an sich bekannten Verfahren hergestellt werden, wobei je nach verwendeten Materialien und deren Funktionsweise unterschiedliche Beschichtungsmethoden angewendet werden können.

Geeignete Beschichtungsverfahren sind zum Beispiel Tauchen, Giessen, Streichen, Rakeln und Schleudergiessen, sowie Aufdampfverfahren, die im Hochvakuum durchgeführt werden. Bei der Anwendung von zum Beispiel Giessverfahren werden im allgemeinen Lösungen in organischen Lösungsmitteln verwendet. Bei der Verwendung von Lösungsmitteln ist darauf zu achten, dass die verwendeten Träger gegen diese Lösungsmittel unempfindlich sind. Geeignete Beschichtungsverfahren sind zum Beispiel in EP-A-0401 791 beschrieben.

Das Aufbringen der metallischen Reflexionsschichten erfolgt bevorzugt durch Aufdampfen im Vakuum. Das aufzubringende Material wird zunächst in ein geeignetes Gefäss gefüllt, das gegebenenfalls mit einer Widerstandsheizung versehen ist, und dann in eine Vakuumkammer gesetzt. Der zu bedampfende Träger wird oberhalb des Gefässes mit dem aufzudampfenden Material in einen Halter eingesetzt. Dieser ist so konstruiert, dass der Träger gegebenenfalls rotiert (z.B. mit 50 U/Min) und geheizt werden kann. Die Vakuumkammer wird auf etwa $1{,}3 \cdot 10^{-5}$ bis $1{,}3 \cdot 10^{-6}$ mbar ($10^{-5}$ bis $10^{-6}$ Torr) evakuiert, und die Heizung wird so eingestellt, dass die Temperatur des aufzudampfenden Materials bis zu dessen Verdampfungstemperatur ansteigt. Die Verdampfung wird so lange fortgesetzt, bis die aufgedampfte Schicht die gewünschte Dicke hat. Je nach Systemaufbau wird zuerst die organische Aufzeichnungsverbindung und dann die reflektierende Schicht aufgebracht, oder es kann umgekehrt verfahren werden. Auf das Aufbringen einer reflektierenden Schicht kann gegebenenfalls verzichtet werden.

Die Sputtertechnik wird besonders wegen der hohen Haftfähigkeit zum Träger für das Aufbringen der metallischen Reflexionsschicht bevorzugt. Das aufzubringende Material (z.B. Aluminium) in Form einer Platte wird als eine "Target"-Elektrode benutzt, während der Träger auf der Gegenelektrode befestigt wird. Zuerst wird die Vakuumkammer auf etwa $10^{-6}$ mbar evakuiert und dann inertes Gas, wie z.B. Argon, bis zu etwa $10^{-3}$ mbar eingeführt. Zwischen der "Target"-Elektrode und der Gegenelektrode wird eine hohe Gleich- oder Radiofrequenz-Spannung von mehreren kV, gegebenenfalls unter Verwendung von Permanentmagneten ("Magnetron-Sputtering"), angelegt, um $Ar^+$-Plasma zu erzeugen. Die durch die $Ar^+$-Ionen von der "Target"-Elektrode gesputterten (herausgeschlagenen) Metallteilchen werden auf dem Substrat gleichmässig und fest deponiert. Die Beschichtung erfolgt innerhalb von einigen 10 Sekunden bis mehreren Minuten je nach Targetmaterialien, Sputtermethode und Sputterbedingungen. Diese Technik ist in Fachbüchern (z.B. W. Kern und L. Vossen, "Thin Film Processes", Academic Press, 1978) ausführlich beschrieben.

Der Aufbau des erfindungsggemässen Aufzeichnungsmaterials richtet sich hauptsächlich nach der Auslesemethode; bekannte Funktionsprinzipien sind die Messung der Veränderung der Transmission oder der Reflexion. Wenn das Aufzeichnungsmaterial gemäss der Veränderung der Lichttransmission aufgebaut wird, kommt zum Beispiel folgende Struktur in Frage: Transparenter Träger/Aufzeichnungsschicht (gegebenenfalls mehrschichtig) und, falls zweckmässig, transparente Schutzschicht. Das Licht zur Aufzeichnung sowie zum Auslesen kann entweder von der Trägerseite oder der Aufzeichnungsschicht bzw. gegebenenfalls der Schutzschichtseite eingestrahlt werden, wobei sich der Lichtdetektor immer auf der Gegenseite befindet.

Wenn das Aufzeichnungsmaterial gemäss der Veränderung der Reflexion aufgebaut wird, so können zum Beispiel folgende andere Strukturen zur Anwendung kommen: transparenter Träger/Aufzeichnungsschicht (gegebenenfalls mehrschichig)/Reflexionsschicht und falls zweckmässig, Schutzschicht (nicht unbedingt transparent), oder Träger (nicht unbedingt transparent)/Reflexionsschicht/Aufzeichnungsschicht und falls zweckmässig transparente Schutzschicht. Im ersten Fall wird das Licht von der Trägerseite eingestrahlt, während im letzen Fall die Strahlung von der Aufzeichnungsschicht- bzw. gegebenenfalls von der Schutzschichtseite einfällt. In beiden Fällen befindet sich der Lichtdetektor auf gleicher Seite wie die Lichtquelle. Der ersterwähnte Aufbau des erfindungsgemäss zu verwendenden Aufzeichnungsmaterials ist im allgemeinen bevorzugt.

Geeignete Laser sind zum Beispiel handelsübliche Diodenlaser, insbesondere Halbleiter-Diodenlaser, zum Beispiel GaAsAl-, InGaAlP- oder GaAs-Laser einer Wellenlänge von 780, 650 beziehungsweise 830 nm, oder He/Ne-Laser (633 nm) und Argon-Laser (514 nm). Die Aufzeichnung kann mit Hilfe eines Lichtmodulators Punkt für Punkt vorgenommen werden.

Das erfindungsgemässe Verfahren ermöglicht eine Speicherung von Informationen mit hoher Zuverlässigkeit und Beständigkeit, welche sich durch eine sehr gute mechanische und thermische Stabilität sowie durch eine hohe Lichtstabilität und scharfe Randzonen auszeichnen. Besonders vorteilhaft ist das überraschend hohe Signal/Rauschverhältnis von Trägermaterial zur Informationsmarkierung, das ein einwandfreies Auslesen zulässt.

Das Auslesen der Information erfolgt durch Messung der Absorption nach dem Reflexionsverfahren oder Transmissionsverfahren unter Verwendung von Laserstrahlung, wobei es besonders vorteilhaft ist, dass Laserstrahlung der zur Aufzeichnung benutzten Wellenlänge verwendet werden kann, also auch kein zweites Lasergerät eingesetzt werden muss. In einer bevorzugten Ausführungsform erfolgt das Aufzeichnen und Auslesen der Information bei gleicher Wellenlänge. Beim Auslesen verwendet man im allgemeinen energiearme Laser, deren Strahlungsintensität gegenüber der zur Aufzeichnung verwendeten Laserstrahlung zum Beispiel um das zehn- bis fünfzigfache reduziert ist. Die Informationen können beim erfindungsgemäss verwendeten Aufzeichnungsmaterial ein- oder mehrfach ausgelesen werden. Die Veränderung im Absorptionsspektrum bzw. die gespeicherte Information kann mit einem Photodetektor unter Verwendung eines energiearmen Lasers abgelesen werden. Geeignete Photodetektoren umfassen PIN-Photodioden sowie Mikroskop-Spektrophotometer (z.B. ®UMSP80 von Carl Zeiss), welche es ermöglichen, die spektralen Veränderungen durch Transmission oder Absorption und insbesondere Reflexion zu messen.

Das erfindungsgemässe Informationen enthaltende Material stellt insbesondere ein optisches Informationsmaterial vom WORM-Typ dar. Es kann zum Beispiel als abspielbare CD (compact disc), als Speichermaterial für Computer oder als Ausweis- und Sicherheitskarte oder für die Herstellung von Hologrammen verwendet werden.

Ein weiterer Gegenstand der Erfindung ist daher die Verwendung der Verbindungen und des Materials zur optischen Aufzeichnung und Speicherung von Information.

Die nachfolgenden Beispiele erläutern die Erfindung näher.

A) Herstellung von Ausgangsprodukten

Beispiel A1: 4-Diphenylphosphanyloxy-phthalodinitril

4,14 g (20 mMol) 4-Brom-phthalodinitril (hergestellt nach A.A. Shapovalov et al, SU 1057491, A1) und 0,12 g (1 mMol) Nickel(II)chlorid werden in einem 25 ml Dreihalskolben, versehen mit Gaseinleitrohr, Vigreux-Kolonne, Destillationsaufsatz und Tropftrichter, unter Rühren und leichtem Argonstrom in einem 170°C heissen Oelbad aufgeschmolzen. Innerhalb von 15 Minuten werden 5,52 g (24 mMol) Diphenylphosphinigsäureethylester zugetropft, und anschliessend 6 Stunden gerührt. Das Reaktionsgemisch wird abgekühlt, mit 100 ml Essigester verdünnt, dreimal mit 25 ml gesättigter NaCl-Lösung gewaschen, über Magnesiumsulfat getrocknet, filtriert und eingedampft. Das braune Oel, welches noch Edukt enthält, wird mittels Flash-Chromatographie (Laufmittel Hexan : Essigsäureethylester = 1 : 1) gereinigt. Man erhält 2,3 g (35% d.Th.) eines klaren farblosen, viskosen Oeles, dessen NMR- und MS-Daten mit der Titelverbindung übereinstimmen.

Beispiel A2: Tetra-(α-2,4-dimethyl-3-pentyloxy)-kupfer-phthalocyanin

In 410 ml Nitrobenzol werden 100,0 g (0,41 Mol) 3-(2,4-Dimethyl-3-pentyloxy)-phthalodinitril, 14,0 g (0,1 Mol) Kupfer(II)chlorid, 49,6 g (0,82 Mol) Harnstoff und 2,0 g (2 Gew.%) Ammoniummolybdat vorgelegt und unter Rühren und Argonatmosphäre auf 160°C erwärmt und anschliessend 5 Stunden bei dieser Temperatur gerührt. Anschliessend wird auf Raumtemperatur abgekühlt, mit Toluol verdünnt und durch Kieselgur filtriert. Das Filtrat wird bei 100°C/$10^{-1}$

mbar vollständig eingedampft. Der Rückstand wird in 1 l Toluol gelöst und mit Toluol als Laufmittel über 600 g Kieselgel filtriert. Das Filtrat wird eingedampft, der Rückstand in 1,5 l Methanol aufgerührt, filtriert, mit Methanol gewaschen und über Nacht bei 60°C/165 mbar getrocknet. Man erhält 99,5 g (94% d.Th.) eines grün-blauen Feststoffes mit einem $\lambda_{max}$ (NMP = N-Methyl-pyrrolidon) von 712 nm ($\varepsilon$ = 197'680 $l \cdot mol^{-1} \cdot cm^{-1}$).

Beispiel A3: Tetra-(α-2,4-dimethyl-3-pentyloxy)-palladium-phthalocyanin

Tetra-(α-2,4-dimethyl-3-pentyloxy)-palladium-phthalocyanin wird analog Beispiel A2 hergestellt, wobei Kupfer(II)chlorid durch Palladium(II)chlorid ersetzt wird.

Beispiel A4: bromiertes Tetra-(α-2,4-dimethyl-3-pentyloxy)-palladium-phthalocyanin)

10 g (9,3 mMol) Tetra-(α-2,4-dimethyl-3-pentyloxy)-palladium-phthalocyanin werden in 100 g Chlorbenzol und 50 g Wasser vorgelegt. Unter Rühren und Argonatmosphäre werden bei 40°C innerhalb von 10 Minuten 7,4 g (46,5 mMol) Brom in 2 g Chlorbenzol zugetropft und anschliessend 1 Stunden bei 60°C gerührt. Das Reaktionsgemisch wird abgekühlt, mit 100 ml Chlorbenzol verdünnt, 1 mal mit 50 ml wässriger 3% $NaHSO_3$ gewaschen, über $MgSO_4$ getrocknet, filtriert und eingedampft. Der Rückstand wird in Toluol gelöst und mit Toluol über 50 g Kieselgel filtriert. Das Filtrat wird auf 80 ml eingedampft und anschliessend auf 700 ml Methanol getropft. Der Niederschlag wird abfiltriert, mit Methanol gewaschen und über Nacht bei 60°C/165 mbar getrocknet. Man erhält 11,6 g (87,9% d.Th.) eines grünen Pulvers mit einem $\lambda_{max}$ = 724 nm (NMP), ($\varepsilon$ = 160'520 $l \cdot mol^{-1} \cdot cm^{-1}$) und einem Bromgehalt von 24,2%. Die Löslichkeit des Produktes beträgt 1,23 g/100ml in Methanol, 1,77 g/100ml in Ethanol, 2,42 g/100ml in 2-Methoxy-ethanol und 9,34 g/100ml in Dibutylether. Das thermische Verhalten wird thermogravimetrisch analysiert, wobei der Zersetzungsbeginn ("onset") bei 282°C liegt, und 20% Gewichtsverlust bei 312°C erreicht werden.

Beispiel A5: bromiertes Tetra-(α-2,4-dimethyl-3-pentyloxy)-kupfer-phthalocyanin

Tetra-(α-2,4-dimethyl-3-pentyloxy)-kupfer-phthalocyanin wird analog Beipiel A4 hergestellt.

Beispiel A6: Mono-β-brom-tris-(α-2,4-dimethyl-pentyloxy)-kupfer-phthalocyanin

1,45 g (6 mMol) 3-(2,4-Dimethyl-3-pentyloxy)phthalodinitril und 0,49 g (2mMol) 4-Brom-phthalodinitril werden mit 0,52 g (2 mMol) Kupfer(II)acetylacetonat, 0,96 g (16 mMol) Harnstoff, 40 mg (2 Gew.%) Ammoniummolybdat in 10 ml Nitrobenzol in einem 50 ml Dreihalskolben, versehen mit Rückflusskühler, Magnetrührer, Thermometer und Argonüberleitung während 4 Stunden unter Rühren auf 150°C erhitzt. Anschliessend wird auf RT abgekühlt, das Reaktionsgemisch auf 50 g Kieselgel in einer Glasfilternutsche gegeben und das Produkt mit Hexan/Essigsäureethylester = 10:1 eluiert. Das Filtrat wird eingedampft und am HV bei 100°C getrocknet. Man erhält 1,1 g (55% d.Th.) eines blauen Feststoffes mit einem Bromgehalt von 7,72%. UV-Spektrum (NMP): $\lambda_{max}$ = 708 nm.

Beispiel A7: Mono-β-brom-tris-(α-2,4-dimethyl-3-pentyloxy)-zinn-phthalocyanin

10,53 g (43,5 mMol) 3-(2,4-Dimethyl-3-pentyloxy)phthalodinitril und 3 g (14,5 mMol) 4-Brom-phthalodinitril werden mit 1,97 g (14,5 mMol) Zink(II)chlorid, 8,8 g (58 mMol) 1,8-Diazabicyclo[5.4.0]undec-7-en (DBU) und 270 ml 1-Pentanol in einem 500 ml Dreihalskolben, versehen mit Rückflusskühler, Magnetrührer, Thermometer und Argonüberleitung während 5,5 Std unter Rühren auf Rückfluss erhitzt. Anschliessend wird auf RT abgekühlt, über ein Filterhilfsmittel filtriert und eingedampft. Der Rückstand wird mit Toluol über 250 g Kieselgel filtriert und die blaue Fraktion eingedampft. Man erhält 7,1 g (49% d.Th.) eines blauen Pulvers mit einem $\lambda_{max}$ = 705 nm (NMP) und einem Bromgehalt von 8,11%.

Beispiel A8: Mono-β-brom-tris-(α-2,4-dimethyl-3-pentyloxy)-phthalocyanin

1,2 l Ethylenglykol-monomethylether werden in einem 2,5 l-Mehrhalskolben mit Thermometer, Ankerrührer, Rückflusskühler und Stickstoffüberleitung vorgelegt und auf 50°C erwärmt. Anschliessend werden unter Inertatmosphäre portionenweise 11,4 g (1,64 Mol) Lithiumpulver zugegeben und gleichzeitig die Reaktionslösung gekühlt, damit die Temperatur nicht über 60°C steigt. Danach werden 30 g (0,123 Mol) 3-(2,4-Dimethyl-3-pentyloxy)phthalodinitril und 8,55 g (41,3 mMol) 4-Brom-phthalodinitril zugegeben und 22 Std bei Rückfluss gerührt. Das Reaktionsgemisch wird auf 40°C abgekühlt, innerhalb von 15 Min 910 ml Essigsäure zugetropft und 30 Min bei dieser Temperatur gerührt. Das Gemisch wird anschliessend eingedampft, und der Rückstand mit Hexan/Essigester = 5:1 über 220 g Kieselgel filtriert und die blaue Fraktion eingedampft. Man erhält 17,0 g (44% d.Th.) eines blauen Pulvers mit einem $\lambda_{max}$ =

731/709 nm (NMP) und einem Bromgehalt von 4,09%.

B) Herstellung erfindungsgemässer Phthalocyanine

Beispiel B1:

Mono-β-diphenylphosphanyloxy-tris-(α-2,4-dimethyl-3-pentyloxy)-kupfer-phthalocyanin

5,1 g (21 mMol) 3-(2,4-Dimethyl-3-pentyloxy)phthalodinitril und 2,3 g (7 mMol) 4-Diphenylphosphanyloxy-phthalodinitril werden mit 1,8 g (7 mMol) Kupfer(II)acetylacetonat, 3,4 g (56 mMol) Harnstoff, 0,15 g (2 Gew.%) Ammoniummolybdat in 20 ml Nitrobenzol in einem 50 ml Dreihalskolben, versehen mit Rückflusskühler, Magnetrührer, Thermometer und Argonüberleitung während 4,5 Stunden unter Rühren auf 155°C erhitzt. Anschliessend wird auf RT abgekühlt, das Reaktionsgemisch auf 100 g Kieselgel in einer Glasfilternutsche gegeben und das restliche Edukt mit Toluol eluiert. Das Produkt wird mit Essigsäureethylester eluiert. Das Filtrat wird eingedampft und am Hochvakuum bei 100°C getrocknet. Der Rückstand wird in Methanol aufgeschlämmt, filtriert und bei 60°C/165 mbar über Nacht getrocknet. Man erhält 2,0 g (26 % d.Th.) eines blauen Feststoffes mit einem Phosphorgehalt von 1,86%. UV-Spektrum (NMP): $\lambda_{max}$ = 709 nm.

Beispiel B2:

Mono-β-diethylphosphonyloxy-tris-(α-2,4-dimethyl-3-pentyloxy)-kupfer-phthalocyanin

0,5 g der Verbindung von Beispiel A6, 56 mg (0,048 mMol) Tetrakis-(triphenylphosphin)-palladium und 50 ml Mesitylen werden in einem 100 ml Dreihalskolben, versehen mit Gaseinleitrohr, Vigreux-Kolonne, Destillationsaufsatz, Thermometer und Tropftrichter, unter Rühren und leichtem Argonstrom auf 155°C erhitzt. Anschliessend werden 0,24 g (1,45 mMol) Triethylphosphit innerhalb von 10 Minuten zugetropft und 3 Stunden gerührt. Danach wird abgekühlt, das Reaktionsgemisch auf 50 g Kieselgel in einer Glasfilternutsche gegeben und das noch vorhandene Edukt mit Toluol eluiert. Das Produkt wird anschliessend mit Essigsäureethylester eluiert. Das Filtrat wird eingedampft und am Hochvakuum bei 100°C getrocknet. Man erhält 0,1 g (20% d.Th.) eines blauen Feststoffes. UV-Spektrum (NMP): $\lambda_{max}$ = 706 nm. Phosphorgehalt = 3,29%.

Beispiel B3:

Mono-β-phenyl-ethylphosphinyloxy-tris-(α-2,4-dimethyl-3-pentyloxy)-kupfer-phthalocyanin

0,5 g der Verbindung von Beispiel A6, 56 mg (0,048 mMol) Tetrakis-(triphenylphosphin)-palladium und 50 ml Mesitylen werden in einem 100 ml Dreihalskolben, versehen mit Gaseinleitrohr, Vigreux-Kolonne, Destillationsaufsatz, Thermometer und Tropftrichter, unter Rühren und leichtem Argonstrom auf 155°C erhitzt. Anschliessend werden 0,29 g (1,45 mMol) Benzolphosphonigsäure-diethylester innerhalb von 10 Minuten zugetropft und 3,5 Stunden gerührt. Danach wird abgekühlt, das Reaktionsgemisch auf 50 g Kieselgel in einer Glasfilternutsche gegeben und das noch vorhandene Edukt mit Toluol eluiert. Das Produkt wird anschliessend mit Essigsäureethylester eluiert. Das Filtrat wird eingedampft und am Hochvakuum bei 100°C getrocknet. Man erhält 0,15 g (30% d.Th.) eines blauen Feststoffes. UV-Spektrum (NMP): $\lambda_{max}$ = 708 nm. Phosphorgehalt = 2,93%.

Beispiel B4:

Mono-β-diphenylphosphanyloxy-tris-(α-2,4-dimethyl-3-pentyloxy)-kupfer-phthalocyanin

5,0 g der Verbindung von Beispiel A6, 620 mg (0,54 mMol) Tetrakis-(triphenylphosphin)-palladium und 120 ml Mesitylen werden in einem 250 ml Dreihalskolben, versehen mit Gaseinleitrohr, Vigreux-Kolonne, Destillationsaufsatz, Thermometer und Tropftrichter, unter Rühren und leichtem Argonstrom auf 160°C erhitzt. Anschliessend werden 3,7 g (16,1 mMol) Diphenylphosphinigsäure-ethylester innerhalb von 20 Minuten zugetropft und 4,5 Stunden bei 180°C gerührt. Danach wird abgekühlt, das Reaktionsgemisch auf 100 g Kieselgel in einer Glasfilternutsche gegeben und das noch vorhandene Edukt mit Toluol eluiert. Das Produkt wird anschliessend mit Essigsäureethylester eluiert. Das Filtrat wird eingedampft, der Rückstand in 100 ml Methanol aufgeschlämmt, filtriert, mit Methanol gewaschen und über Nacht bei 60°C/165 mbar getrocknet. Man erhält 1,0 g (20% d.Th.) eines blauen Feststoffes. UV-Spektrum (NMP): $\lambda_{max}$ = 709 nm. Phosphorgehalt = 3,16%.

Beispiel B5: diethylphosphonato-haltiges, bromiertes Tetra-(α-2,4-dimethyl-3-pentyloxy)-palladium-phthalocyanin

2 g bromiertes Tetra-(α-2,4-dimethyl-3-pentyloxy)-palladium-phthalocyanin (Bromgehalt = 18,2%), hergestellt analog Beispiel A4, 59 mg (0,46 mMol) Nickel(II)chlorid und 50 ml Mesitylen werden in einem 100 ml Dreihalskolben, versehen mit Gaseinleitrohr, Vigreux-Kolonne, Destillationsaufsatz, Thermometer und Tropftrichter, unter Rühren und leichtem Argonstrom auf 170°C Oelbadtemperatur erhitzt. Anschliessend werden 1,14 g (6,84 mMol) Triethylphosphit innerhalb von 20 Minuten zugetropft und 5 Stunden gerührt. Danach wird abgekühlt, das Reaktionsgemisch auf 60 g Kieselgel in einer Glasfilternutsche gegeben und das noch vorhandene Edukt mit Toluol eluiert. Das Produkt wird anschliessend mit Essigsäureethylester eluiert. Das Filtrat wird eingedampft und am Hochvakuum bei 120°C getrocknet. Man erhält 0,64 g (30% d.Th.) eines blau-grünen Feststoffes. UV-Spektrum (NMP): $\lambda_{max}$ 706 nm. Phosphorgehalt = 2,50%, Bromgehalt = 10,8%.

Beispiel B6: diethylphosphonato-haltiges, bromiertes Tetra-(α-2,4-dimethyl-3-pentyloxy)-palladium-phathalocyanin

2 g bromiertes Tetra-(α-2,4-dimethyl-3-pentyloxy)-palladium-phthalocyanin (Bromgehalt = 22,5%), hergestellt analog Beispiel A4, 0,32 g (0,28 mMol) Tetrakis-(triphenylphosphin)-palladium, 0,85 g (6,19 mMol) Diethylphosphit, 0,63 g (6,19 mMol) Triethylamin und 50 ml Mesitylen werden in einem 100 ml Dreihalskolben, versehen mit Thermometer, Rückflusskühler, Stickstoffüberleitung und Magnetrührer, unter Rühren und leichtem Argonstrom auf 90°C erhitzt. und 24 Stunden gerührt. Danach wird abgekühlt, das Reaktionsgemisch auf 60 g Kieselgel in einer Glasfilternutsche gegeben und das noch vorhandene Edukt mit Toluol eluiert. Das Produkt wird anschliessend mit Hexan/Essigsäureethylester = 1:1 eluiert. Das Filtrat wird eingedampft und der Rückstand über Nacht bei 60°C/165 mbar getrocknet. Man erhält 0,33 g (16% d.Th.) eines blau-grünen Feststoffes. UV-Spektrum (NMP): $\lambda_{max}$ = 720 nm. Phosphorgehalt = 2,13%.

Beispiel B7: diethylphosphonato-haltiges, bromiertes Tetra-(α-2,4-dimethyl-3-pentyloxy)-palladium-phthalocyanin

2 g bromiertes Tetra-(α-2,4-dimethyl-3-pentyloxy)-palladium-phthalocyanin (Bromgehalt = 28,5%), hergestellt analog Beispiel A4, 12 mg (0,072 mMol) Palladiumdichlorid, 75 mg (0,288 mMol) Triphenylphosphin und 50 ml Dimethylformamid werden in einem 100 ml Dreihalskolben, versehen mit Thermometer, Septum, Rückflusskühler, Stickstoffüberleitung und Magnetrührer, unter Rühren und leichtem Argonstrom auf Rückfluss erhitzt. Innerhalb von 15 Minuten werden 3,56 g (21,45 mMol) Triethylphosphit zugetropft; anschliessend wird 4 Stunden am Rückfluss gehalten. Danach wird abgekühlt und am Rotationsverdampfer eingedampft. Der Rückstand wird in Toluol gelöst, auf 60 g Kieselgel in einer Glasfilternutsche gegeben und das noch vorhandene Edukt mit Hexan/Essigsäureethylester = 25:1 eluiert. Das Produkt wird anschliessend mit Hexan/Essigsäureethylester = 1:1 eluiert. Das Filtrat wird eingedampft und der Rückstand über Nacht bei 60°C/165 mbar getrocknet. Man erhält 1,2 g (57% d.Th.) eines amorphen blau-grünen Feststoffes. UV-Spektrum (NMP): $\lambda_{max}$ = 721 nm. Das thermische Verhalten wird thermogravimetrisch analysiert, wobei der Zersetzungsbeginn ("onset") bei 265°C liegt, und 20% Gewichtsverlust bei 294°C erreicht werden. Die Löslichkeit des Produktes beträgt 1,45 g/100ml in Methanol, 4,64 g/100ml in Ethanol, 13,15 g/100ml in 2-Methoxy-ethanol und 10,73 g/100ml in Dibutylether.

Beispiel B8: diethylphosphonato-haltiges, bromiertes Tetra-(α-2,4-dimethyl-3-pentyloxy)-palladium-phthalocyanin

12,5 g bromiertes Tetra-(α-2,4-dimethyl-3-pentyloxy)-palladium-phthalocyanin (Bromgehalt = 23,4%), hergestellt analog Beispiel A4, 80 mg (0,45 mMol) Palladiumdichlorid, 472 mg (1,8 mMol) Triphenylphosphin und 140 ml Dimethylformamid werden in einem 250 ml Dreihalskolben, versehen mit Thermometer, Septum, Rückflusskühler, Stickstoffüberleitung und Magnetrührer, unter Rühren und leichtem Argonstrom auf Rückfluss erhitzt. Innerhalb von 15 Minuten werden 18,2 g (105 mMol) Triethylphosphit zugetropft; anschliessend wird 6½ Stunden am Rückfluss gehalten. Danach wird abgekühlt und am Rotationsverdampfer eingedampft. Dem in 50 ml Methylenchlorid gelösten Rückstand werden 12 Kieselgel zugegeben. Das Gemisch wird eingedampft und der Rückstand auf 100 g Kieselgel in einer Glasfiltemutsche aufgeschichtet. Das noch vorhandene Edukt wird mit Hexan/Essigsäureethylester = 25:1 eluiert. Das Produkt wird anschliessend mit Hexan/Essigsäureethylester = 1:1 eluiert. Das Filtrat wird eingedampft und der Rückstand über Nacht bei 60°C/165 mbar getrocknet. Man erhält 10 g eines amorphen blau-grünen Feststoffes, welches in 50 ml Tetrahydrofuran gelöst und durch Zutropfen von 75 ml Wasser gefällt wird. Das umgefällte Produkt wird 5x mit je 100 ml Wasser gewaschen und über Nacht bei 60°C/30-40 mbar getrocknet. Man erhält 8,83 g eines dunkelgrünen Pulvers. UV-Spektrum (NMP): $\lambda_{max}$ = 721 nm ($\varepsilon$ = 149'450 $l \cdot mol^{-1} \cdot cm^{-1}$). Phosphorgehalt = 2,91%, Bromgehalt = 14,15%.

Beispiel B9: phenylethylphosphinato-haltiges, bromiertes Tetra-(α-2,4-dimethyl-3-pentyloxy)-palladium-phthalocyanin

2 g bromiertes Tetra-(α-2,4-dimethyl-3-pentyloxy)-palladium-phthalocyanin (Bromgehalt = 18,2%), hergestellt analog Beispiel A4, 94 mg (0,73 mMol) Nickel(II)chlorid und 20 ml Mesitylen werden in einem 100 ml Dreihalskolben, versehen mit Gaseinleitrohr, Vigreux-Kolonne, Destillationsaufsatz, Thermometer und Tropftrichter, unter Rühren und leichtem Argonstrom auf 160°C Oelbadtemperatur erhitzt. Anschliessend werden 0,98 g (5,02 mMol) Benzolphosphonigsäure-diethylester innerhalb von 20 Minuten zugetropft und 4,75 Stunden gerührt. Danach wird abgekühlt, und das Reaktionsgemisch bei 120°C am Hochvakuum eingedampft. Der Rückstand wird in Toluol gelöst, auf 100 g Kieselgel in einer Glasfilternutsche gegeben und das noch vorhandene Edukt mit Toluol eluiert. Das Produkt wird anschliessend mit Essigsäureethylester eluiert. Das Filtrat wird eingedampft und am Hochvakuum bei 120°C getrocknet. Man erhält 0,3 g (15% d.Th.) eines blau-grünen Feststoffes. UV-Spektrum (NMP): $\lambda_{max}$ = 708 nm. Phosphorgehalt = 3,25%, Bromgehalt = 7,94%.

Beispiel B10: diphenylphosphinoxy-haltiges, bromiertes Tetra-(α-2,4-dimethyl-3-pentyloxy)-palladium-phthalocyanin

2 g bromiertes Tetra-(α-2,4-dimethyl-3-pentyloxy)-palladium-phthalocyanin (Bromgehalt = 19,1%), hergestellt analog Beispiel A4, 0,55 g (0,48 mMol) Tetrakis-(triphenylphosphin)-palladium und 50 ml Mesitylen werden in einem 100 ml Dreihalskolben, versehen mit Gaseinleitrohr, Vigreux-Kolonne, Destillationsaufsatz, Thermometer und Tropftrichter, unter Rühren und leichtem Argonstrom auf 180°C Oelbadtemperatur erhitzt. Anschliessend werden 3,3 g (14,34 mMol) Diphenylphosphinigsäure-ethylester innerhalb von 10 Minuten zugetropft und 4 Stunden gerührt. Danach wird abgekühlt, auf 100 g Kieselgel in einer Glasfilternutsche gegeben und das noch vorhandene Edukt mit Toluol eluiert. Das Produkt wird anschliessend mit Essigsäureethylester eluiert. Das Filtrat wird eingedampft und am Hochvakuum bei 120°C getrocknet. Man erhält 0,2 g (10% d.Th.) eines blau-grünen Feststoffes. UV-Spektrum (NMP): $\lambda_{max}$ = 718 nm. Phosphorgehalt = 2,43%, Bromgehalt = 12,81%.

Beispiel B11: diethylphosphonato-haltiges, bromiertes Tetra-(α-2,4-dimethyl-3-pentyloxy)-kupfer-phthalocyanin

4 g bromiertes Tetra-(α-2,4-dimethyl-3-pentyloxy)-kupfer-phthalocyanin (Bromgehalt = 16,6%), hergestellt analog Beispiel A5, 0,96 g (0,831 mMol) Tetrakis-(triphenylphosphin)-palladium und 100 ml Mesitylen werden in einem 250 ml Dreihalskolben, versehen mit Gaseinleitrohr, Vigreux-Kolonne, Destillationsaufsatz, Thermometer und Tropftrichter, unter Rühren und leichtem Argonstrom auf 180°C Oelbadtemperatur erhitzt. Anschliessend werden 4,14 g (24,93 mMol) Triethylphosphit innerhalb von 20 Minuten zugetropft und 2,5 Stunden gerührt. Danach wird abgekühlt, und das Mesitylen bei 100°C am Hochvakuum eingedampft. Der Rückstand wird in Toluol gelöst, auf 100 g Kieselgel in einer Glasfilternutsche gegeben und das noch vorhandene Edukt mit Toluol eluiert. Das Produkt wird anschliessend mit Essigsäureethylester eluiert. Das Filtrat wird eingedampft und am Hochvakuum bei 120°C getrocknet. Man erhält 2,6 g (65% d.Th.) eines blau-grünen Feststoffes. UV-Spektrum (NMP): $\lambda_{max}$ = 721 nm. Phosphorgehalt = 3,23%, Bromgehalt = 8,72%.

Beispiel B12: phosphonsäure-bis-(diethylamid)-haltiges, bromiertes Tetra-(α-2,4-dimethyl-3-pentyloxy)-palladium-phthalocyanin

2 g bromiertes Tetra-(α-2,4-dimethyl-3-pentyloxy)-palladium-phthalocyanin (Bromgehalt = 22,5%), hergestellt analog Beispiel A4, 0,65 g (0,56 mMol) Tetrakis-(triphenylphosphin)-palladium und 50 ml Mesitylen werden in einem 100 ml Dreihalskolben, versehen mit Gaseinleitrohr, Vigreux-Kolonne, Destillationsaufsatz, Thermometer und Tropftrichter, unter Rühren und leichtem Argonstrom auf 180°C Oelbadtemperatur erhitzt. Anschliessend werden 3,5 g (16,89 mMol) Phosphorigsäure-ethylester-bis-(diethylamid) innerhalb von 30 Minuten zugetropft und 4 Stunden gerührt. Danach wird abgekühlt, auf 100 g Kieselgel in einer Glasfilternutsche gegeben und das noch vorhandene Edukt mit Toluol eluiert. Das Produkt wird anschliessend mit Essigsäureethylester eluiert. Das Filtrat wird eingedampft und der Rückstand bei 60°C/165 mbar getrocknet. Man erhält 0,15 g (8% d.Th.) eines blau-grünen Feststoffes. UV-Spektrum (NMP): $\lambda_{max}$ = 722 nm.
Phosphorgehalt = 4,25%, Bromgehalt = 15,89%.

Beispiel B13: diphenylphosphino-haltiges, bromiertes Tetra-(α-2,4-dimethyl-3-pentyloxy)-palladium-phthalocyanin

In einem 50 ml-Dreihalskolben mit Stickstoffüberleitung, Rückflusskühler, Thermometer und Magnetrührerwerden 1,48 g (5,63 mMol) Triphenylphosphin, 0,12 g (16,9 mMol) Lithiumpulver und 15 ml Tetrahydrofuran (frisch absolutiert über Na) vorgelegt und 3 h bei RT gerührt. Anschliessend werden 0,52 g (5,63 mMol) t-Butylchlorid zugegeben und die orange-rote Suspension 40 Minuten auf Rückfluss erhitzt. Danach wird abgekühlt und die überstehende

Lösung in einen 100ml-Dreihalskolben mit Thermometer, Rückflusskühler, Tropftrichter, Stickstoffüberleitung und Magnetrührer übergeführt und wieder auf Rückfluss erhitzt. Innerhalb von 20 Minuten werden 2 g bromiertes Tetra-(α-2,4-dimethyl-3-pentyloxy)-palladium-phthalocyanin (Bromgehalt = 22,5%), hergestellt analog Beispiel A4, gelöst in 20 ml abs. THF zugetropft und 2 h reagieren lassen. Danach wird abgekühlt und das Lösungsmittel eingedampft. Der Rückstand wird in Toluol gelöst, mit Wasser gewaschen und über Magnesiumsulfat getrocknet. Die Lösung wird filtriert und auf 100 g Kieselgel in einer Glasfilternutsche gegeben und das Produkt mit Toluol eluiert. Das Filtrat wird eingedampft und der Rückstand über Nacht bei 60°C/165 mbar getrocknet. Man erhält 1,8 g (90% d.Th.) eines blau-grünen Feststoffes. UV-Spektrum (NMP): $\lambda_{max}$ = 722 nm. Phosphorgehalt = 5,72%, Bromgehalt = 6,52%.

Beispiel B14:

Mono-β-diethylphosphonyloxy-tris-(α-2,4-diemethyl-3-pentyloxy)-zink-phthalocyanin

7,0 g (7 mMol) der Verbindung von Beispiel A7, 62 mg (0,35 mMol) Palladium(II)chlorid, 0,37 g (1,4 mMol) Triphenylphosphin und 175 ml Dimethylformamid werden einem 250 ml Dreihalskolben, versehen mit Gaseinleitrohr, Vigreux-Kolonne, Destillationsaufsatz, Thermometer, Magnetrührer und Tropftrichter, unter Rühren und leichtem Argonstrom auf 155°C erhitzt. Anschliessend werden 3,53 g (21,3 mMol) Triethylphosphit innerhalb von 20 Min zugetropft und 8 Std gerührt. Danach wird abgekühlt und das Reaktionsgemisch eingedampft. Der Rückstand wird auf 100 g Kieselgel in einer Glasfilternutsche gegeben und das noch vorhandene Edukt mit Hexan/Essigester = 25:1 eluiert. Das Produkt wird dann mit Hexan/Essigester = 1:1 eluiert. Das Filtrat wird eingedampft. Man erhält 1,5 g (20% d.Th.) eines blauen Feststoffes. UV (NMP): $\lambda_{max}$ = 707 nm. Phosphorgehalt = 3,59%.

Beispiel B15:

Mono-β-diethylphosphonyloxy-tris-(α-2,4-diemethyl-3-pentyloxy)-phthalocyanin

17,0 g (18,2 mMol) der Verbindung von Beispiel A8, 160 mg (0,91 mMol) Palladium(II)-chlorid, 0,95 g (3,63 mMol) Triphenylphosphin und 400 ml Dimethylformamid werden einem 1 l Dreihalskolben, versehen mit Gaseinleitrohr, Vigreux-Kolonne, Destillationsaufsatz, Thermometer, Magnetrührer und Tropftrichter, unter Rühren und leichtem Argonstrom auf 155°C erhitzt. Anschliessend werden 4,33 g (26,1 mMol) Triethylphosphit innerhalb von 25 Min zugetropft und 11 Std gerührt. Danach wird abgekühlt und das Reaktionsgemisch eingedampft. Der Rückstand wird auf 220 g Kieselgel in einer Glasfilternutsche gegeben und das noch vorhandene Edukt mit Hexan/Essigester = 25:1 eluiert. Das Produkt wird dann mit Hexan/Essigester = 1:1 eluiert. Das Filtrat wird eingedampft. Man erhält 2,4 g (13,3% d.Th.) eines blauen Feststoffes. UV (NMP): $\lambda_{max}$ = 723 nm. Phosphorgehalt = 3,19%.

C) Anwendungsbeispiele

Beispiel C1: Optisches Aufzeichnungsmaterial

Von der gemäss Beispiel B6 hergestellten Verbindung wird eine 2%ige Lösung in Methylcyclohexan hergestellt und nach vollständiger Auflösung über ein 0,2 µm Teflonfilter filtriert. Die Farbstofflösung wird anschliessend bei 150 U/Min während 8 Sekunden auf eine 1,2 mm dicke gerillte Polycarbonatplatte aufgeschleudert ("spin coating"-Verfahren). Die Drehzahl wird auf 1200 U/Min erhöht, wobei überschüssige Farbstofflösung abgeschleudert wird und eine gleichmässige Feststoffschicht entsteht. Die Polycarbonatplatte mit der Aufzeichnungsschicht wird während 10 Min bei 60°C getrocknet. Auf die Aufzeichnungsschicht wird eine 14 µm dicke Reflexionsschicht aus Gold in einer Vakuumbedampfungsanlage aufgedampft. Darüber wird mittels Aufschleuderneine 13 µm dicke UV-härtbare acrylathaltige Polymerschutzschicht der Firma Dainippon Ink (®Daicure SD-17) aufgebracht und mittels Belichtung gehärtet. In die Aufzeichnungsschicht werden mit einer Halbleiterdiode, die bei 780 nm emittiert, mit einer Leistung von 8 mW bei einer linearen Geschwindigkeit von 1,2 bis 1,4 m/s Signale gemäss dem EFM-CD Format (Eight to Fourteen Modulation Compact Disc Format) eingeschrieben. Die so aufgezeichneten Signale haben Längen von 0,9 µm bis 3,3 µm bei Intervallen von 0,8 µm bis 3,5 µm und zeigen ein den Vorschriften entsprechendes Eye Diagramm. Anschliessend werden die aufgezeichneten Signale auf einem handelsüblichen CD-Wiedergabegerät mit einer Leistung 0,5 mW bei 780 nm ausgelesen. Das Aufzeichnungsmaterial hat eine sehr gute Wiedergabequalität.

**Patentansprüche**

1. Phthalocyanin oder dessen Metallkomplex von einem divalenten Metall oder einem divalenten Oxometall, dadurch

gekennzeichnet, dass es mindestens einen phosphorhaltigen Substituenten über das Phosphoratom am peripheren Kohlenstoffgerüst gebunden enthält.

**2.** Verbindung nach Anspruch 1, dadurch gekennzeichnet, dass sie der allgemeinen Strukturformel $(G)_k$-A-$(Q)_l$ entspricht, wobei A ein Phthalocyanin oder sein Metallkomplex mit einem divalenten Metall oder divalenten Oxometall bedeutet, G für phosphorhaltige Substituenten und Q für weitere Substituenten steht, wobei k eine Zahl von 1 bis 16, bevorzugt von 1 bis 4, und l unabhängig davon 0 oder eine Zahl von 1 bis 15 bedeuten, mit der Massgabe, dass die Summe von k und l eine Zahl kleiner oder gleich 16, bevorzugt von 4 bis 16, ergibt.

**3.** Verbindung nach Anspruch 1, dadurch gekennzeichnet, dass der phosphorhaltige Substituent aus der Gruppe bestehend aus $-PR_1R_2$, $-P(X)R_3$, -P(X)(Y), $-PZR_4R_5$, $-PZ(X)R_6$, -PZ(X)(Y) ausgewählt ist, wobei

$R_1$, $R_2$, $R_3$, $R_4$, $R_5$ und $R_6$ unabhängig voneinander unsubstituiertes oder mit -OH, Halogen, $C_1$-$C_{12}$-Alkoxy, $-COOR_7$, -CN, Phenyl, Naphthyl substituiertes lineares oder verzweigtes $C_1$-$C_{12}$-Alkyl, $C_3$-$C_{12}$-Alkenyl oder $C_3$-$C_{12}$-Alkinyl, unsubstituiertes oder mit -OH, Halogen, $C_1$-$C_{12}$-Alkoxy, $-COOR_7$, -CN substituiertes $C_3$-$C_8$-Cycloalkyl, Phenyl oder Naphthyl, bevorzugt lineares oder verzweigtes unsubstituiertes $C_1$-$C_{12}$-Alkyl, $C_3$-$C_{12}$-Alkenyl, $C_3$-$C_{12}$-Alkinyl, $C_3$-$C_8$-Cycloalkyl, Phenyl oder Naphthyl, besonders bevorzugt lineares oder verzweigtes unsubstituiertes $C_1$-$C_8$-Alkyl, $C_3$-$C_8$-Alkenyl, $C_3$-$C_8$-Alkinyl, $C_3$-$C_8$-Cycloalkyl oder Phenyl, bedeuten;

X und Y unabhängig voneinander $-OR_8$, $-SR_9$ oder $-NR_{10}R_{11}$ sind;

Z für O, S, Se oder Te, bevorzugt für O oder S steht;

$R_7$ lineares oder verzweigtes $C_1$-$C_{12}$-Alkyl, $C_3$-$C_{12}$-Alkenyl oder $C_3$-$C_{12}$-Alkinyl, bevorzugt lineares oder verzweigtes unsubstituiertes $C_1$-$C_{12}$-Alkyl, ist;

$R_8$, $R_9$ unabhängig voneinander ein Alkalimetall-Kation, $NH_4^+$, Wasserstoff, unsubstituiertes oder mit -OH, Halogen, $C_1$-$C_{12}$-Alkoxy, $-COOR_7$, -CN, Phenyl, Naphthyl substituiertes lineares oder verzweigtes $C_1$-$C_{12}$-Alkyl, $C_3$-$C_{12}$-Alkenyl oder $C_3$-$C_{12}$-Alkinyl, unsubstituiertes oder mit -OH, Halogen, $C_1$-$C_{12}$-Alkoxy, $-COOR_7$, -CN substituiertes $C_3$-$C_8$-Cycloalkyl, Phenyl oder Naphthyl, bevorzugt ein Alkalimetall-Kation, $NH_4^+$, Wasserstoff, lineares oder verzweigtes unsubstituiertes $C_1$-$C_{12}$-Alkyl, $C_3$-$C_{12}$-Alkenyl oder $C_3$-$C_{12}$-Alkinyl, $C_3$-$C_8$-Cycloalkyl oder Phenyl, besonders bevorzugt $C_1$-$C_6$-Alkyl, $C_3$-$C_8$-Cycloalkyl oder Phenyl, sind; und

$R_{10}$, $R_{11}$ unabhängig voneinander Wasserstoff oder unsubstituiertes oder mit -OH, Halogen, $C_1$-$C_{12}$-Alkoxy, $-COOR_7$, -CN, Phenyl, Naphthyl substituiertes lineares oder verzweigtes $C_1$-$C_{12}$-Alkyl, $C_3$-$C_{12}$-Alkenyl oder $C_3$-$C_{12}$-Alkinyl bedeuten, oder unsubstituiertes oder mit -OH, Halogen, $C_1$-$C_{12}$-Alkoxy, $-COOR_7$, -CN substituiertes $C_3$-$C_8$-Cycloalkyl, Phenyl oder Naphthyl, bevorzugt lineares oder verzweigtes unsubstituiertes $C_1$-$C_{12}$-Alkyl, $C_3$-$C_{12}$-Alkenyl oder $C_3$-$C_{12}$-Alkinyl, $C_3$-$C_8$-Cycloalkyl oder Phenyl, besonders bevorzugt lineares oder verzweigtes unsubstituiertes $C_1$-$C_6$-Alkyl, sind.

**4.** Phthalocyanin-Metallkomplex nach Anspruch 1, dadurch gekennzeichnet, dass das divalente Metall Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II) oder Pb(II), oder das divalente Oxometall V(O), MnO oder TiO, bevorzugt Zn(II), Sn(II), Cu(II), Ni(II), Co(II), Pb(II) oder Pd(II) oder das divalente Oxometall V(O), ist.

**5.** Verbindung nach Anspruch 1 entsprechend der Formel I,

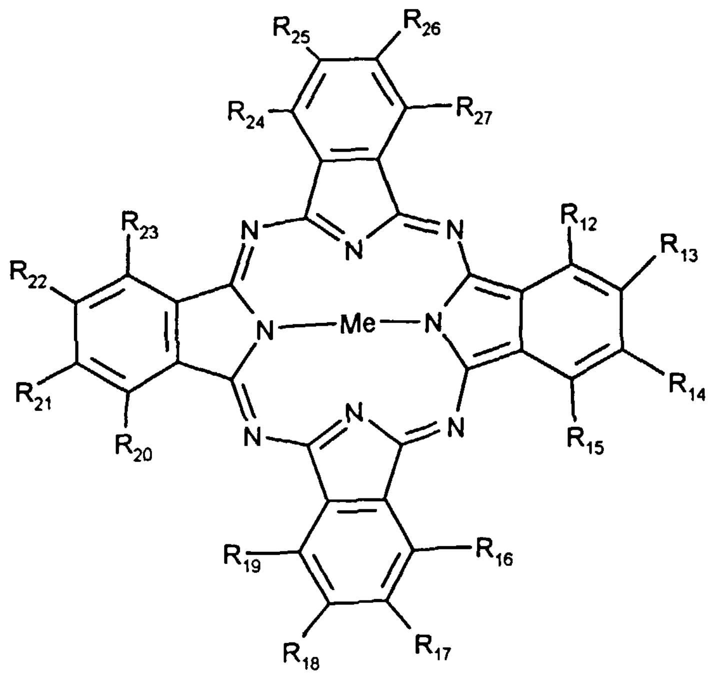

(I)

worin Me ein divalentes Metallatom oder ein divalentes, Oxometall oder 2 Wasserstoffatome bedeutet,

$R_{12}$ bis $R_{27}$ unabhängig voneinander $-PR_1R_2$, $-P(X)R_3$, -P(X)(Y), $-PZR_4R_5$, $-PZ(X)R_6$, -PZ(X)(Y), Wasserstoff, -OH, Halogen, unsubstituiertes oder mit -OH, Halogen, $C_1$-$C_{12}$-Alkoxy, $-COOR_7$, -CN, Phenyl, Naphthyl substituiertes lineares oder verzweigtes $C_1$-$C_{12}$-Alkyl, $C_3$-$C_{12}$-Alkenyl, $C_3$-$C_{12}$-Alkinyl, $C_1$-$C_{12}$-Alkoxy, oder $C_1$-$C_{12}$-Alkylthio, unsubstituiertes oder mit -OH, Halogen, $C_1$-$C_{12}$-Alkoxy, $-COOR_7$, -CN substituiertes $C_3$-$C_8$-Cycloalkyl, Phenyl, Naphthyl, Phenoxy, Thiophenyl, Naphthoxy oder Thionaphthyl, $-COOR_{28}$, $-CONR_{29}R_{30}$, $-SO_3R_{31}$, $-SO_2NR_{32}R_{33}$, $-SiR_{34}R_{35}R_{36}$, $-NR_{37}R_{38}$ bedeuten, worin

$R_{28}$ bis $R_{38}$ unabhängig voneinander Wasserstoff, unsubstituiertes oder mit -OH, Halogen, $-COOR_7$, -CN, Phenyl, Naphthyl substituiertes lineares oder verzweigtes $C_1$-$C_{12}$-Alkyl, $C_3$-$C_{12}$-Alkenyl, $C_3$-$C_{12}$-Alkinyl, unsubstituiertes oder mit -OH, Halogen, $C_1$-$C_{12}$-Alkoxy, $-COOR_7$, -CN substituiertes $C_3$-$C_8$-Cycloalkyl, Phenyl, Naphthyl sind;

mit der Maßgabe, dass mindestens einer der Substituenten $R_{12}$ bis $R_{27}$ ein phosphorhaltiger Substituent, ausgewählt aus der Gruppe, bestehend aus $-PR_1R_2$, $-P(X)R_3$, -P(X)(Y), $-PZR_4R_5$, $-PZ(X)R_6$, -PZ(X)(Y) ist, wobei $R_1$-$R_6$, X, Y und Z die im Anspruch 3 angegebene Bedeutung haben.

**6.** Verbindung nach Anspruch 5, dadurch gekennzeichnet, dass $R_{12}$ bis $R_{27}$ unabhängig voneinander Wasserstoff, Halogen, lineares oder verzweigtes unsubstituiertes $C_1$-$C_{12}$-Alkyl, $C_3$-$C_{12}$-Alkenyl, $C_3$-$C_{12}$-Alkinyl, $C_1$-$C_{12}$-Alkoxy, $C_1$-$C_{12}$-Alkylthio, Phenyl, Phenoxy oder Thiophenyl, bevorzugt Wasserstoff, Halogen, lineares oder verzweigtes unsubstituiertes $C_4$-$C_{10}$-Alkyl, $C_4$-$C_{10}$-Alkenyl, $C_4$-$C_{10}$-Alkinyl oder $C_4$-$C_{10}$-Alkoxy, besonders bevorzugt Wasserstoff, Br, Cl oder lineares oder verzweigtes unsubstituiertes $C_4$-$C_{10}$-Alkoxy, sind.

**7.** Verbindung nach Anspruch 5, dadurch gekennzeichnet, dass $R_{28}$ bis $R_{38}$ unabhängig voneinander Wasserstoff, lineares oder verzweigtes unsubstituiertes $C_1$-$C_{12}$-Alkyl, $C_3$-$C_{12}$-Alkenyl, oder $C_3$-$C_{12}$-Alkinyl, $C_3$-$C_8$-Cycloalkyl, Phenyl oder Naphthyl, bevorzugt Wasserstoff oder $C_1$-$C_{12}$-Alkyl, sind.

**8.** Verbindung nach Anspruch 5, dadurch gekennzeichnet, dass

$R_1$ bis $R_6$ unabhänig voneinander $C_1$-$C_4$-Alkyl oder Phenyl sind;
X und Y unabhängig voneinander $-OR_8$ oder $-NR_{10}R_{11}$ bedeuten;
Z für O steht;
Me Pd, V(O), Cu oder Ni ist; und

$R_{12}$ bis $R_{27}$ unabhänig voneinander Wasserstoff, Br oder lineares oder verzweigtes unsubstituiertes $C_4$-$C_{10}$-Alkoxy bedeuten.

**9.** Phthalocyanin-Metallkomplex nach Anspruch 1, dadurch gekennzeichnet, dass die phosphorhaltigen Substituenten Reste der Formel II-VI

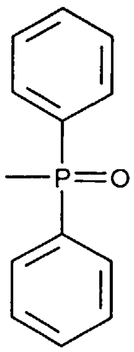 (II), 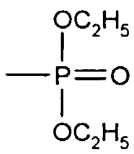 (III), 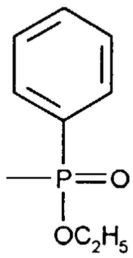 (IV), 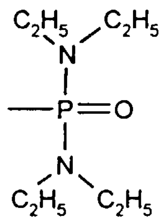 (V), 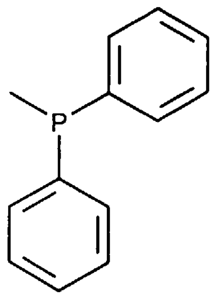 (VI),

darstellen.

**10.** Verbindung nach Anspruch 5, worin Me für Pd steht und die Reste $R_{12}$ bis $R_{27}$ Wasserstoff, Brom, 2,4-Dimethyl-3-pentyloxy oder $-PO(OC_2H_5)_2$ sind, wobei 3 der Reste $R_{12}$ bis $R_{27}$ Brom, 2 der Reste $R_{12}$ bis $R_{27}$ $-PO(OC_2H_5)_2$, 4 der Reste $R_{12}$, $R_{15}$, $R_{16}$, $R_{19}$, $R_{20}$, $R_{23}$, $R_{24}$ und $R_{27}$ 2,4-Dimethyl-3-pentyloxy, und die übrigen 7 Reste $R_{12}$ bis $R_{27}$ Wasserstoff sind.

**11.** Verbindung nach Anspruch 5, worin Me für Cu steht und die Reste $R_{12}$ bis $R_{27}$ Wasserstoff, Brom, 2,4-Dimethyl-3-pentyloxy oder $-PO(OC_2H_5)_2$ sind, wobei 1 oder 2 der Reste $R_{12}$ bis $R_{27}$ Brom, 1 der Reste $R_{12}$ bis $R_{27}$ $-PO(OC_2H_5)_2$, 4 der Reste $R_{12}$, $R_{15}$, $R_{16}$, $R_{19}$, $R_{20}$, $R_{23}$, $R_{24}$ und $R_{27}$ 2,4-Dimethyl-3-pentyloxy, und die übrigen 10 oder 9 Reste $R_{12}$ bis $R_{27}$ Wasserstoff sind.

**12.** Verfahren zur Herstellung einer Verbindung nach Anspruch 1 durch Umsetzung eines einen phosphorhaltigen Substituenten enthaltenden Phthalodinitrils oder Diiminodihydroisoindolins, oder eines Gemisches von Phthalodinitrilen oder Diiminodihydroisoindolinen, von welchen mindestens eines einen phosphorhaltigen Substituenten enthält, welche gegebenenfalls unabhängig voneinander weitere Substituenten enthalten können, gegebenenfalls in Gegenwart eines Metallsalzes oder Oxometallsalzes.

**13.** Verfahren nach Anspruch 12 zur Herstellung einer Verbindung der Formel I nach Anspruch 5 durch Umsetzung einer Verbindung der Formel VII oder VIII

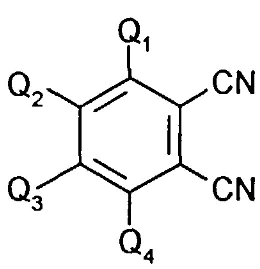 (VII), 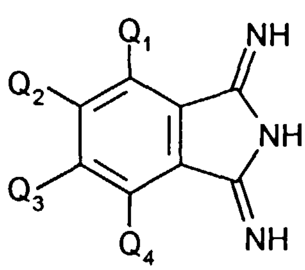 (VIII),

oder eines Gemisches von 2 bis 4 dieser Verbindungen,
gegebenenfalls in Gegenwart eines Metallsalzes, wobei $Q_1$ bis $Q_4$ unabhängig voneinander $-PR_1R_2$, $-P(X)R_3$, $-P(X)(Y)$, $-PZR_4R_5$, $-PZ(X)R_6$, $-PZ(X)(Y)$, Wasserstoff, -OH, Halogen, unsubstituiertes oder mit -OH, Halogen, $C_1$-$C_{12}$-Alkoxy, $-COOR_7$, -CN, Phenyl, Naphthyl substituiertes $C_1$-$C_{12}$-Alkyl, $C_3$-$C_{12}$-Alkenyl, $C_3$-$C_{12}$-Alkinyl, $C_1$-$C_{12}$-Alkoxy, oder

$C_1$-$C_{12}$-Alkylthio, unsubstituiertes oder mit -OH, Halogen, $C_1$-$C_{12}$-Alkoxy, -$COOR_7$, -CN substituiertes $C_3$-$C_8$-Cycloalkyl, Phenyl, Naphthyl, Phenoxy, Thiophenyl, Naphthoxy oder Thionaphthyl, -$COOR_{28}$, -$CONR_{29}R_{30}$, -$SO_3R_{31}$, -$SO_2NR_{32}R_{33}$, -$SiR_{34}R_{35}R_{36}$, -$NR_{37}R_{38}$ bedeuten, worin
$R_1$ bis $R_7$ und $R_{28}$ bis $R_{38}$ die vorstehend angegebene Bedeutung haben,
mit der Maßgabe, dass mindestens eine Verbindung der Formel VII oder VIII mindestens einen Substituent $Q_1$ bis $Q_4$ trägt, der aus der Gruppe phosphorhaltigen Substituent bestehend aus -$PR_1R_2$, -$P(X)R_3$, -P(X)(Y), -$PZR_4R_5$, -$PZ(X)R_6$, -PZ(X)(Y) ausgewählt ist, wobei $R_1$-$R_6$, X, Y und Z die im Anspruch 3 angegebene Bedeutung haben.

**14.** Verfahren zur Herstellung einer Verbindung nach Anspruch 1 durch Umsetzung eines Phthalocyanins oder seines Metallkomplexes von divalenten Metallen oder divalenten Oxometallen, der mindestens einen Halogensubstituenten als Abbgangsgruppe enthält, in Gegenwart eines Metallkatalysators mit einer phosphorhaltigen Verbindung.

**15.** Verfahren nach Anspruch 14, dadurch gekennzeichnet, dass die phosphorhaltige Verbindung ausgewählt wird aus der Gruppe, bestehend aus $PR_1R_2R_3$, $P(X)_2R_3$, $P(X)_2(Y)$, $P(X)R_1R_2$, $P(X)_2OH$, wobei $R_1$ bis $R_6$, X, Y und Z die in Anspruch 3 angegebene Bedeutung haben.

**16.** Verfahren nach Anspruch 14, dadurch gekennzeichnet, dass die Umsetzung in Gegenwart eines Palladium(II)-Komplexes in einem polaren Lösungsmittel mit einer Dielektrizitätskonstante $\varepsilon \geq 25$, bevorzugt in einem Amid, besonders bevorzugt in Dimethylformamid, durchgeführt wird.

**17.** Verfahren nach Anspruch 16, worin der Palladium(II)-Komplex aus $PdCl_2$ und Triphenylphosphin erzeugt wird.

**18.** Material zur optischen Aufzeichnung und Speicherung von Information, bei dem auf einem transparenten, dielektrischen Trägermaterial (1) mindestens eine Schicht (2) einer Verbindung nach Anspruch 1 aufgebracht ist.

**19.** Verwendung einer Verbindung nach Anspruch 1 zur optischen Aufzeichnung und Speicherung von Information.

## Claims

**1.** Phthalocyanine or a metal complex thereof with a divalent metal or a divalent oxo metal which contains at least one phosphorus-containing substituent bonded to the peripheral carbon skeleton via the phosphorus atom.

**2.** A compound according to claim 1, which conforms to the general structural formula $(G)_k$-A-$(Q)_l$, in which A is a phthalocyanine or a metal complex thereof with a divalent metal or a divalent oxo metal, G is a phosphorus-containing substituent, Q is a further substituent, k is a number from 1 to 16, preferably from 1 to 4, and 1, independently thereof, is 0 or a number from 1 to 15, with the proviso that the sum of k and 1 is a number less than or equal to 16, preferably from 4 to 16.

**3.** A compound according to claim 1, wherein the phosphorus-containing substituent is selected from the group consisting of -$PR_1R_2$, -$P(X)R_3$, -P(X)(Y), -$PZR_4R_5$, -$PZ(X)R_6$, and -PZ(X)(Y), where

$R_1$, $R_2$, $R_3$, $R_4$, $R_5$ and $R_6$, independently of one another, are linear or branched $C_1$-$C_{12}$alkyl, $C_3$-$C_{12}$alkenyl or $C_3$-$C_{12}$alkynyl, which is unsubstituted or substituted by -OH, halogen, $C_1$-$C_{12}$alkoxy, -$COOR_7$, -CN, phenyl or naphthyl, or are $C_3$-$C_8$cycloalkyl, phenyl or naphthyl which is unsubstituted or substituted by -OH, halogen, $C_1$-$C_{12}$alkoxy, -$COOR_7$ or -CN, preferably linear or branched, unsubstituted $C_1$-$C_{12}$alkyl, $C_3$-$C_{12}$alkenyl, $C_3$-$C_{12}$alkynyl, $C_3$-$C_8$cycloalkyl, phenyl or naphthyl, particularly preferably linear or branched, unsubstituted $C_1$-$C_8$alkyl, $C_3$-$C_8$alkenyl, $C_3$-$C_8$alkynyl, $C_3$-$C_8$cycloalkyl or phenyl;

X and Y, independently of one another, are -$OR_8$, -$SR_9$ or -$NR_{10}R_{11}$;

Z is O, S, Se or Te, preferably O or S;

$R_7$ is linear or branched $C_1$--$C_{12}$alkyl, $C_3$-$C_{12}$alkenyl or $C_3$-$C_{12}$alkynyl, preferably linear or branched, unsubstituted $C_1$-$C_{12}$alkyl;

$R_8$ and $R_9$, independently of one another, are an alkali metal cation, $NH_4^+$, hydrogen, linear or branched $C_1$-$C_{12}$alkyl, $C_3$-$C_{12}$alkenyl or $C_3$-$C_{12}$alkynyl, which is unsubstituted or substituted by -OH, halogen, $C_1$-$C_{12}$alkoxy,

-$COOR_7$, -CN, phenyl or naphthyl, or are $C_3$-$C_8$cycloalkyl, phenyl or naphthyl which is unsubstituted or substituted by -OH, halogen, $C_1$-$C_{12}$alkoxy, -$COOR_7$ or -CN, preferably an alkali metal cation, $NH_4^+$, hydrogen, linear or branched, unsubstituted $C_1$-$C_{12}$alkyl, $C_3$-$C_{12}$alkenyl or $C_3$-$C_{12}$alkynyl, $C_3$-$C_8$cycloalkyl or phenyl, particularly preferably $C_1$-$C_6$alkyl, $C_3$-$C_8$cycloalkyl or phenyl and

$R_{10}$, and $R_{11}$, independently of one another, are hydrogen, linear or branched $C_1$-$C_{12}$alkyl, $C_3$-$C_{12}$alkenyl or $C_3$-$C_{12}$alkynyl which is unsubstituted or substituted by -OH, halogen, $C_1$-$C_{12}$alkoxy, -$COOR_7$, -CN, phenyl or naphthyl, or are $C_3$-$C_8$cycloalkyl, phenyl or naphthyl which is unsubstituted or substituted by OH, halogen, $C_1$-$C_{12}$alkoxy, $COOR_7$ or CN, preferably linear or branched, unsubstituted $C_1$-$C_{12}$alkyl, $C_3$-$C_{12}$alkenyl or $C_3$-$C_{12}$alkynyl, $C_3$-$C_8$cycloalkyl or phenyl, particularly preferably linear or branched, unsubstituted $C_1$-$C_6$alkyl.

4. Phthalocyanine/metal complex according to claim 1, wherein the divalent metal is Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II) or Pb(II), or the divalent oxo metal is V(O), MnO or TiO, preferably Zn(II), Sn(II), Cu(II), Ni(II), Co(II), Pb(II) or Pd(II) or the divalent oxo metal is V(O).

5. A compound according to claim 1, conforming to the formula I,

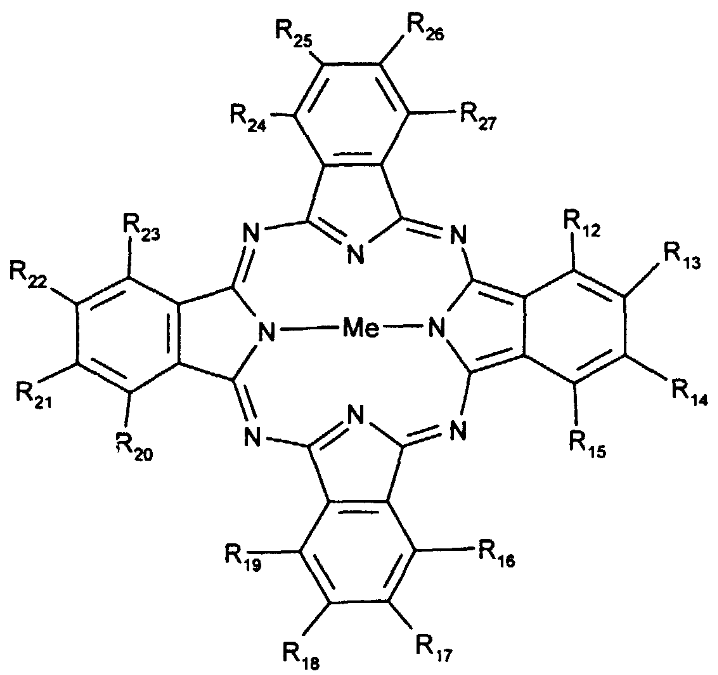

(I)

in which Me is a divalent metal atom or a divalent oxo metal or 2 hydrogen atoms,
$R_{12}$ to $R_{27}$ are, independently of one another, -$PR_1R_2$, -$P(X)R_3$, -P(X)(Y), -$PZR_4R_5$, -$PZ(X)R_6$, -PZ(X)(Y), hydrogen, -OH, halogen, linear or branched $C_1$-$C_{12}$alkyl, $C_3$-$C_{12}$alkenyl, $C_{3-12}$alkynyl, $C_1$-$C_{12}$alkoxy, or $C_1$-$C_{12}$alkylthio which is unsubstituted or substituted by OH, halogen, $C_1$-$C_{12}$alkoxy, $COOR_7$, CN, phenyl or naphthyl, $C_3$-$C_8$cycloalkyl, phenyl, naphthyl, phenoxy, thiophenyl, naphthoxy or thionaphthyl which is unsubstituted or substituted by OH, halogen, $C_1$-$C_{12}$alkoxy, $COOR_7$ or CN, or are -$COOR_{28}$, -$CONR_{29}R_{30}$, -$SO_3R_{31}$, -$SO_2NR_{32}R_{33}$, -$SiR_{34}R_{35}R_{36}$ or -$NR_{37}R_{38}$, where
$R_{28}$ to $R_{38}$, independently of one another, are hydrogen,
linear or branched $C_1$-$C_{12}$alkyl, $C_3$-$C_{12}$alkenyl or $C_3$-$C_{12}$alkynyl which is unsubstituted or substituted by OH, halogen, $COOR_7$, CN, phenyl or naphthyl, or are $C_3$-$C_8$cycloalkyl, phenyl or naphthyl which is unsubstituted or substituted by OH, halogen, $C_1$-$C_{12}$alkoxy, $COOR_7$ or CN;

with the proviso that at least one of the substituents $R_{12}$ to $R_{27}$ is a phosphorus-containing substituent selected from the group consisting of -$PR_1R_2$, -$P(X)R_3$, -P(X)(Y), -$PZR_4R_5$, -$PZ(X)R_6$, -PZ(X)(Y) , where $R_1$-$R_6$, X, Y and Z are as defined in claim 3.

**6.** A compound according to claim 5, where $R_{12}$ to $R_{27}$, independently of one another, are hydrogen, halogen, linear or branched, unsubstituted $C_1$-$C_{12}$alkyl, $C_3$-$C_{12}$alkenyl, $C_3$-$C_{12}$alkynyl, $C_1$-$C_{12}$alkoxy, $C_1$-$C_{12}$alkylthio, phenyl, phenoxy or thiophenyl, preferably hydrogen, halogen, linear or branched, unsubstituted $C_4$-$C_{10}$alkyl, $C_4$-$C_{10}$alkenyl, $C_4$-$C_{10}$alkynyl or $C_4$-$C_{10}$alkoxy, particularly preferably hydrogen, Br, Cl or linear or branched, unsubstituted $C_4$-$C_{10}$alkoxy.

**7.** A compound according to claim 5, wherein $R_{28}$ to $R_{38}$, independently of one another, are hydrogen, linear or branched, unsubstituted $C_1$-$C_{12}$alkyl, $C_3$-$C_{12}$alkenyl, or $C_3$-$C_{12}$alkynyl, $C_3$-$C_8$cycloalkyl, phenyl or naphthyl, preferably hydrogen or $C_1$-$C_{12}$alkyl.

**8.** A compound according to claim 5, wherein

$R_1$ to $R_6$, independently of one another, are $C_1$-$C_4$alkyl or phenyl;
X and Y, independently of one another, are $-OR_8$ or $-NR_{10}R_{11}$;
Z is O;
Me is Pd, VO, Cu or Ni; and
$R_{12}$ to $R_{27}$, independently of one another, are hydrogen, Br or linear or branched, unsubstituted $C_4$-$C_{10}$alkoxy.

**9.** A phthalocyanine/metal complex according to claim 1, wherein the phosphorus- containing substituents are radicals of the formulae II-VI

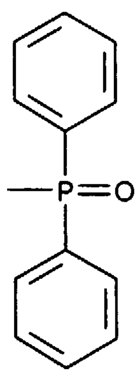 (II), 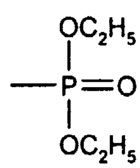 (III), 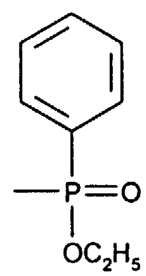 (IV), 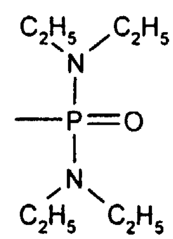

(V), 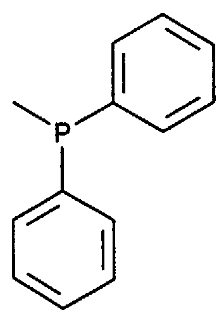 (VI).

**10.** A compound according to claim 5, in which Me is Pd, and the radicals $R_{12}$ to $R_{27}$ are hydrogen, bromine, 2,4-dimethyl-3-pentoxy or $-PO(OC_2H_5)_2$, where 3 of the radicals $R_{12}$ to $R_{27}$ are bromine, 2 of the radicals $R_{12}$ to $R_{27}$ are $-PO(OC_2H_5)_2$, 4 of the radicals $R_{12}$, $R_{15}$, $R_{16}$, $R_{19}$, $R_{20}$, $R_{23}$, $R_{24}$ and $R_{27}$ are 2,4-dimethyl-3-pentoxy, and the other 7 radicals $R_{12}$ to $R_{27}$ are hydrogen.

**11.** A compound according to claim 5, in which Me is Cu, and the radicals $R_{12}$ to $R_{27}$ are hydrogen, bromine, 2,4-dimethyl-3-pentoxy or $-PO(OC_2H_5)_2$, where 1 or 2 of the radicals $R_{12}$ to $R_{27}$ are bromine, 1 of the radicals $R_{12}$ to $R_{27}$ is $-PO(OC_2H_5)_2$, 4 of the radicals $R_{12}$, $R_{15}$, $R_{16}$, $R_{19}$, $R_{20}$, $R_{23}$, $R_{24}$ and $R_{27}$ are 2,4-dimethyl-3-pentoxy, and the other 10 or 9 radicals $R_{12}$ to $R_{27}$ are hydrogen.

**12.** A process for the preparation of a compound according to claim 1 by reacting a phthalonitrile or diiminodihydroisoindoline which contains a phosphorus-containing substituent, or a mixture of phthalodinitriles or diiminodihydroisoindolines at least one of which contains a phosphorus-containing substituent, and which if desired, independently of one another, may contain further substituents, if desired in the presence of a metal salt or oxo metal salt.

**13.** A process according to claim 12 for the preparation of a compound of the formula I according to claim 5 by reacting a compound of the formula VII or VIII

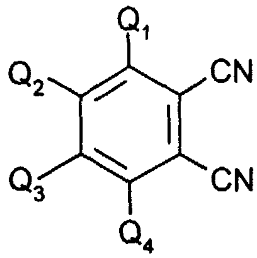

(VII),

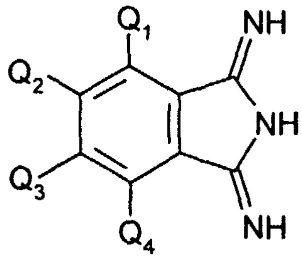

(VIII),

or a mixture of 2 to 4 of these compounds,
if desired in the presence of a metal salt, where $Q_1$ to $Q_4$, independently of one another, are $-PR_1R_2$, $-P(X)R_3$, -P (X)(Y), $-PZR_4R_5$, $-PZ(X)R_6$, -PZ(X)(Y), hydrogen, OH, halogen, $C_1$-$C_{12}$alkyl, $C_3$-$C_{12}$alkenyl, $C_3$-$C_{12}$alkynyl, $C_1$-$C_{12}$alkoxy, or $C_1$-$C_{12}$alkylthio which is unsubstituted or substituted by OH, halogen, $C_1$-$C_{12}$alkoxy, $COOR_7$, CN, phenyl or naphthyl, $C_3$-$C_8$cycloalkyl, phenyl, naphthyl, phenoxy, thiophenyl, naphthoxy or thionaphthyl which is unsubstituted or substituted by OH, halogen, $C_1$-$C_{12}$alkoxy, $COOR_7$ or CN, or are $-COOR_{28}$, $-CONR_{29}R_{30}$, $-SO_3R_{31}$, $-SO_2NR_{32}R_{33}$, $-SiR_{34}R_{35}R_{36}$ or $-NR_{37}R_{38}$, in which $R_1$ to $R_7$ and $R_{28}$ to $R_{38}$ are as defined above,
with the proviso that at least one compound of the formula VII or VIII carries at least one substituent $Q_1$ to $Q_4$ selected from the group of phosphorus-containing substituents consisting of $-PR_1R_2$, $-P(X)R_3$, -P(X)(Y), $-PZR_4R_5$, $-PZ(X)R_6$, -PZ(X)(Y), where $R_1$-$R_6$, X, Y and Z are as defined in claim 3.

**14.** A process for the preparation of a compound according to claim 1 by reacting a phthalocyanine or a metal complex thereof with divalent metals or divalent oxo metals containing at least one halogen substituent as leaving group, with a phosphorus-containing compound in the presence of a metal catalyst.

**15.** A process according to claim 14, wherein the phosphorus-containing compound is selected from the group consisting of $PR_1R_2R_3$, $P(X)_2R_3$, $P(X)_2(Y)$, $P(X)R_1R_2$, $P(X)_2OH$, where $R_1$ to $R_6$, X, Y and Z are as defined in claim 3.

**16.** A process according to claim 14, wherein the reaction is carried out in the presence of a palladium(II) complex in a polar solvent having a dielectric constant $\varepsilon$ of $\geq 25$, preferably in an amide, particularly preferably in dimethylformamide.

**17.** A process according to claim 16, in which the palladium(II) complex is produced from $PdCl_2$ and triphenylphosphine.

**18.** A material for the optical recording and storage of information, in which at least one layer (2) of a compound according to claim 1 has been applied to a transparent, dielectric carrier material (1).

**19.** The use of a compound according to claim 1 for the optical recording and storage of information.

## Revendications

**1.** Phtalocyanine ou son complexe métallique avec un métal bivalent ou un oxométal bivalent, caractérisée en ce qu'elle présente au moins un substituant phosphoré relié au squelette de carbone périphérique par l'intermédiaire

de l'atome de phosphore.

**2.** Composé selon la revendication 1, caractérisé en ce que il répond à la formule développé générale $(G)_x$-A-$(Q)_l$, où A représente une phtalocanine ou son complexe métallique avec un métal bivalent ou un oxométal bivalent, G représente des substituants phosphorés et Q représente d'autres substituants, k étant un nombre de 1 à 16, de préférence de 1 à 4, et 1 indépendamment vaut 0 ou représente un nombre de 1 à 15, à la condition que la somme de k et l soit un nombre inférieur ou égal à 16, de préférence de 4 à 16.

**3.** Composé selon la revendication 1, caractérisé en ce que le substituant phosphoré est pris dans le groupe constitué par $-PR_1R_2$, $-P(X)R_3$, -P(X)(Y), $PZR_4R_5$, $-PZ(X)R_6$, PZ(X)(Y),

$R_1$, $R_2$, $R_3$, $R_4$, $R_5$ et $R_6$ représentant, indépendamment les uns des autres, des groupes alkyle en $C_1$-$C_{12}$, alcényle en $C_3$-$C_{12}$ ou alcynyle en $C_3$-$C_{12}$, linéaires ou ramifiés, non substitués ou substitués, par des substituants -OH, halogène, alkoxy en $C_1$-$C_{12}$, $-COOR_7$, -CN, phényle, naphtyle, des groupes cycloalkyle en $C_3$-$C_8$, phényle ou naphtyle non substitués ou substitués par des substituants -OH, halogène, alkoxy en $C_1$-$C_{12}$, $-COOR_7$, -CN, de préférence cycloalkyle en $C_3$-$C_8$, phényle, naphtyle, alkyle en $C_1$-$C_{12}$, alcényle en $C_3$-$C_{12}$, alcynyle en $C_3$-$C_{12}$, linéaires ou ramifiés, non substitués, de manière particulièrement préférée des groupes cycloalkyle en $C_3$-$C_8$, phényle, alkyle en $C_1$-$C_8$, alcényle en $C_3$-$C_8$, alcynyle en $C_3$-$C_8$, linéaires ou ramifiés, non substitués ;
X et Y représentent, indépendamment l'un de l'autre, $-OR_8$, $-SR_9$ ou $-NR_{10}R_{11}$ ;
Z représente des atomes de O, S, Se ou Te, de préférence O ou S ;
$R_7$ représente des groupes alkyle en $C_1$-$C_{12}$, alcényle en $C_3$-$C_{12}$ ou alcynyle en $C_3$-$C_{12}$ linéaires ou ramifiés, de préférence alkyle en $C_1$-$C_{12}$ linéaire ou ramifié non substitué ;
$R_8$, $R_9$ représentent, indépendamment l'un de l'autre, des cations de métaux alcalins, $NH_4^+$, des atomes d'hydrogène, des groupes alkyle en $C_1$-$C_{12}$, alcényle en $C_3$-$C_{12}$ ou alcynyle en $C_3$-$C_{12}$ linéaires ou ramifiés, non substitués ou substitués par des substituants -OH, halogène, alkoxy en $C_1$-$C_{12}$, $-COOR_7$, -CN, phényle, naphtyle, des groupes cycloalkyle en $C_3$-$C_8$, phényle ou naphtyle non substitués ou substitués par des substituants -OH, halogène, alkoxy en $C_1$-$C_{12}$, $-COOR_7$, -CN, de préférence un cation de métal alcalin, $NH_4^+$, un atome d'hydrogène, des groupes cycloalkyle en $C_3$-$C_8$, phényle, alkyle en $C_1$-$C_{12}$, alcényle en $C_3$-$C_{12}$ ou alcynyle en $C_3$-$C_{12}$ linéaires ou ramifiés, non substitués, de manière particulièrement préfère, alkyle en $C_1$-$C_6$, cycloalkyle en $C_3$-$C_8$ ou phényle ; et
$R_{10}$, $R_{11}$ représentent, indépendamment l'un de l'autre, des atomes d'hydrogène ou des groupes alkyle en $C_1$-$C_{12}$, alcényle en $C_3$-$C_{12}$ ou alcynyle en $C_3$-$C_{12}$, linéaires ou ramifiés, non substitués ou substitués par des substituants -OH, halogène, alkoxy en $C_1$-$C_{12}$, $-COOR_7$, -CN, phényle, naphtyle, ou représentent des groupes cycloalkyle en $C_3$-$C_8$, phényle ou naphtyle non substitués ou substitués par des substituants -OH, halogène, alkoxy en $C_1$-$C_{12}$, $-COOR_7$, -CN, de préférence des groupes cycloalkyle en $C_3$-$C_8$, phényle, alkyle en $C_1$-$C_{12}$, alcényle en $C_3$-$C_{12}$ ou alcynyle en $C_3$-$C_{12}$, linéaires ou ramifiés, non substitués, de manière particulièrement préférée, alkyle en $C_1$-$C_6$ linéaire ou ramifié.

**4.** Complexe métallique de phtalocynine selon la revendication 1, caractérisé en ce que le métal bivalent est Cu(II), Zn(II), Fe(II), Ni(II), Ru(II), Rh(II), Pd(II), Pt(II), Mn(II), Mg(II), Be(II), Ca(II), Ba(II), Cd(II), Hg(II), Sn(II), Co(II) ou Pb(II), ou l'oxométal bivalent V(O), MnO ou TiO, de préférence Zn(II), Sn(II), Cu(II), Ni(II), Co(II), Pb(II) ou Pd(II), ou l'oxométal bivalent est V(O).

**5.** Composé selon la revendication 1 répondant à la formule I,

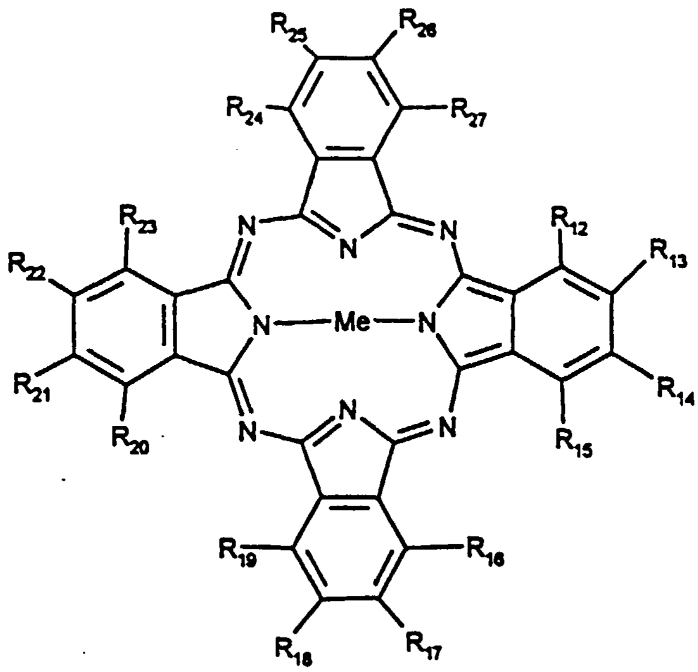

(I)

où

Me représente un atome métallique bivalent ou un oxométal bivalent ou 2 atomes d'hydrogène,

$R_{12}$ à $R_{27}$ représentent, indépendamment les uns des autres, $-PR_1R_2$, $-P(X)R_3$, -P(X)(Y), $PZR_4R_5$, $-PZ(X)R_6$, PZ(X)(Y), des atomes d'hydrogène, des groupes -OH, des atomes d'halogène, des groupes alkyle en $C_1$-$C_{12}$, alcényle en $C_3$-$C_{12}$, alcynyle en $C_3$-$C_{12}$, alkoxy en $C_1$-$C_{12}$ ou (alkyl en $C_1$-$C_{12}$)thio linéaires ou ramifiés, non substitués ou substitués par des substituants -OH, halogène, alkoxy en $C_1$-$C_{12}$, $-COOR_7$, -CN, phényle, naphtyle, des groupes cycloalkyle en $C_3$-$C_8$, phényle, naphtyle, phénoxy, thiophényle, naphtoxy, ou thionaphtyle non substitués ou substitués par des substituants -OH, halogène, alkoxy en $C_1$-$C_{12}$, $-COOR_7$, -CN, des groupes $-COOR_{28}$, $-COONR_{29}R_{30}$, $-SO_3R_{31}$, $-SO_2NR_{32}R_{33}$, $-SiR_{34}R_{35}R_{36}$, $-NR_{37}R_{38}$, où $R_{28}$ à $R_{38}$ représentent, indépendamment les uns des autres, des atomes d'hydrogène, des groupes alkyle en $C_1$-$C_{12}$, alcényle en $C_3$-$C_{12}$ ou alcynyle en $C_3$-$C_{12}$ linéaires ou ramifiés, non substitués ou substitués par des substituants -OH, halogène, -$COOR_7$, -CN, phényle, naphtyle, des groupes cycloalkyle en $C_3$-$C_8$, phényle ou naphtyle non substitués ou substitués par des substituants -OH, halogène, alkoxy en $C_1$-$C_{12}$, $-COOR_7$, -CN ;

à la condition qu'au moins l'un des substituants $R_{12}$ à $R_{27}$ soit un substituant phosphoré pris dans le groupe constitué par $-PR_1R_2$, $-P(X)R_3$, -P(X)(Y), $PZR_4R_5$, $-PZ(X)R_6$, PZ(X)(Y), la signification de $R_1$ à $R_6$, X, Y et Z ainsi que leurs préférences étant donnée à la revendication 3.

**6.** Composé selon la revendication 5, caractérisé en ce que $R_{12}$ à $R_{27}$ représentent, indépendamment l'un de l'autre, des atomes d'hydrogène, des atomes d'halogènes, des groupes des groupes alkyle en $C_1$-$C_{12}$, alcényle en $C_3$-$C_{12}$, alcynyle en $C_3$-$C_{12}$, alkoxy en $C_1$-$C_{12}$ ou (alkyl en $C_1$-$C_{12}$)thio, phényle, phénoxy ou thiophényle linéaires ou ramifiés non substitués, de préférence des atomes d'hydrogène, des atomes d'halogènes, des groupes alkyle en $C_4$-$C_{10}$, alcényle en $C_4$-$C_{10}$, alcynyle en $C_4$-$C_{10}$, alkoxy en $C_4$-$C_{10}$ linéaires ou ramifiésnon substitués, de manière particulièrement préférée des atomes d'hydrogène, de Br, de Cl ou des groupes alkoxy en $C_4$-$C_{10}$ linéaires ou ramifiés non substitués.

**7.** Composé selon la revendication 5, caractérisé en ce que $R_{28}$ à $R_{38}$, indépendamment les uns des autres, représentent des atomes d'hydrogène, des groupes cycloalkyle en $C_3$-$C_8$, phényle, naphtyle, alkyle en $C_1$-$C_{12}$, alcényle en $C_3$-$C_{12}$ ou alcynyle en $C_3$-$C_{12}$ linéaires ou ramifiés, non substitués, de préférence des atomes d'hydrogène ou des groupes alkyle en $C_1$-$C_{12}$.

**8.** Composé selon la revendication 5, caractérisé en ce que

$R_1$ à $R_4$ représentent, indépendamment les uns des autres, des groupes alkyle en $C_1$-$C_4$ ou phényle,
X et Y représentent, indépendamment l'un de l'autre, -$OR_8$ ou $NR_{10}R_{11}$ ;
Z représente un atome de O ;
Me représente des atomes de Pd, V(O), Cu ou Ni ; et
$R_{12}$ à $R_{27}$ représentent, indépendamment les uns des autres, des atomes d'hydrogène, de Br ou des groupes alkoxy en $C_4$-$C_{10}$ linéaires ou ramifiés, non substitués.

**9.** Complexe métallique de phtalocyanine selon la revendication 1, caractérisé en ce que les subtituants phosphorés représentent des restes de formule II à IV

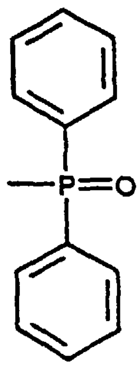 (II), 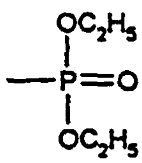 (III), 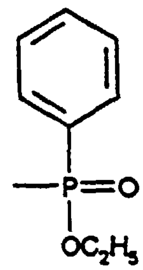 (IV), 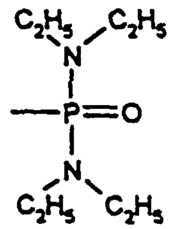 (V), 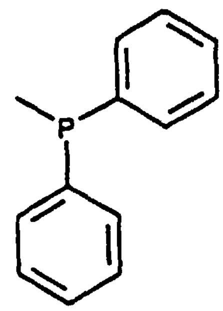 (VI).

**10.** Composé selon la revendication 5, où Me représente un atome de Pd et les restes $R_{12}$ à $R_{27}$ représentent des atomes d'hydrogène, des atomes de brome, des groupes 2,4-diméthyl-3-pentyloxy ou -$PO(OC_2H_5)_2$, 3 des restes $R_{12}$ à $R_{27}$ représentent des atomes de brome, 2 des restes $R_{12}$ à $R_{27}$ représentent des groupes -$PO(OC_2H_5)_2$, 4 des restes $R_{12}$, $R_{15}$, $R_{16}$, $R_{19}$, $R_{20}$, $R_{23}$, $R_{24}$ et $R_{27}$ représentent des groupes 2,4-diméthyl-3-pentyloxy et les autres 7 restes $R_{12}$ à $R_{27}$ représentent des atomes d'hydrogène.

**11.** Composé selon la revendication 5, où Me représente un atome de Cu et les restes $R_{12}$ à $R_{27}$ représentent des atomes d'hydrogène, des atomes de brome, des groupes 2,4-diméthyl-3-pentyloxy ou -$PO(OC_2H_5)_2$, où 1 ou 2 des restes $R_{12}$ à $R_{27}$ représentent des atomes de brome, 1 des restes $R_{12}$ à $R_{27}$ représente -$PO(OC_2H_5)_2$, 4 des restes $R_{12}$, $R_{15}$, $R_{16}$, $R_{19}$, $R_{20}$, $R_{23}$, $R_{24}$ et $R_{27}$ représentent 2,4-diméthyl-3-pentyloxy et les autres 10 ou 9 restes $R_{12}$ à $R_{27}$ représentent des atomes d'hydrogène.

**12.** Procédé de préparation d'un composé selon la revendication 1, par réaction d'un phtalodinitrile ou d'une diiminodihydroisoindoline présentant un substituant phosphoré, ou d'un mélange de phtalo-dinitriles ou de diiminodihydroisoindolines, dont au moins un présente un substituant phosphoré, lesquels peuvent présenter, indépendamment les uns des autres, d'autres substituants, éventuellement en présence d'un sel métallique ou d'un sel d'oxométal.

**13.** Procédé selon la revendication 12 pour la préparation d'un composé de formule I selon la revendication 5, par réaction d'un composé de formule VII ou VIII

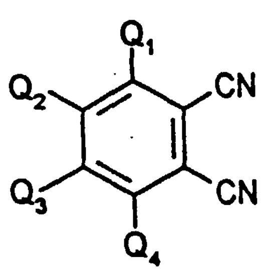 (VII), 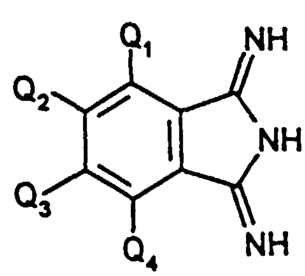 (VIII),

ou d'un mélange de 2 à 4 de ces composés,

éventuellement en présence d'un sel métallique, où

| | |
|---|---|
| $Q_1$ à $Q_4$ | représentant, indépendamment les uns des autres, $-PR_1R_2$, $-P(X)R_3$, -P(X)(Y), $PZR_4R_5$, $-PZ(X)R_6$, PZ(X)(Y), des atomes d'hydrogène, des groupes -OH, des atomes d'halogène, des groupes alkyle en $C_1$-$C_{12}$, alcényle en $C_3$-$C_{12}$, alcynyle en $C_3$-$C_{12}$, alkoxy en $C_1$-$C_{12}$ ou (alkyl en $C_1$-$C_{12}$)thio non substitués ou substitués par des substituants -OH, halogène, alkoxy en $C_1$-$C_{12}$, $-COOR_7$, -CN, phényle, naphtyle, des groupes cycloalkyle en $C_3$-$C_8$, phényle, naphtyle, phénoxy, thiophényle, naphtoxy, ou thionaphtyle non substitués ou substitués par des substituants -OH, halogène, alkoxy en $C_1$-$C_{12}$, $-COOR_7$, -CN, des groupes $-COOR_{28}$, $-COONR_{29}R_{30}$, $-SO_3R_{31}$, $-SO_2NR_{32}R_{33}$, $-SiR_{34}R_{35}R_{36}$, $-NR_{37}R_{38}$, où |
| $R_1$ à $R_7$ et $R_{28}$ à $R_{38}$ | possèdent la signification donnée auparavant, |

à la condition qu'au moins un composé de formule VII ou VIII porte au moins un substituant $Q_1$ à $Q_4$, pris dans le groupe des substituants phosphorés constitué par $-PR_1R_2$, $-P(X)R_3$, -P(X)(Y), $PZR_4R_5$, $-PZ(X)R_6$, PZ(X)(Y), où $R_1$ à $R_6$, X, Y et Z possèdent la signification donnée à la revendication 3.

**14.** Procédé de préparation d'un composé selon la revendication 1 par transformation d'une phtalocyanine ou de son complexe métallique de métaux bivalents ou d'oxométaux bivalents, présentant au moins un substituant halogéné en tant que groupe partant, en présence d'un catalyseur métallique avec un composé phosphoré.

**15.** Composé selon la revendication 14, caractérisé en ce que le composé phosphoré est pris dans le groupe constitué par $-PR_1R_2R_3$, $-P(X)_2R_3$, $-P(X)_2(Y)$, $P(X)R_1R_2$, $-P(X)_2OH$, $R_1$ à $R_6$, X, Y et Z possédant la signification donnée à la revendication 3.

**16.** Procédé selon la revendication 14, caractérisé en ce qu'on met en oeuvre la réaction en présence d'un complexe de palladium(II) dans un solvant polaire ayant une constante diélectrique de $\varepsilon \geq 25$, de préférence dans un amide, de manière particulièrement préférée dans le diméthylformamide.

**17.** Procédé selon la revendication 16, où le complexe de palladium(II) est produit à partir de $PdCl_2$ et la triphénylphosphine.

**18.** Matière pour enregistrement et stockage optiques d'informations, où on applique sur une matière de support diélectrique, transparente (1) au moins une couche (2) d'un composé selon la revendication 1.

**19.** Utilisation d'un composé selon la revendication 1 pour enregistrement et stockage optiques d'informations.